# EUROPEAN PATENT APPLICATION

(11) **EP 4 834 883 A1**
(43) Date of publication of application: **04.11.2026**
(21) Application number: 23962964.5
(22) Date of filing: 30.12.2023
(51) Int. Cl.: C07D 221/14, A61K 31/473, A61P 31/18

(54) **HNRNPA2B1 AGONIST COMPOUND AND USE THEREOF IN TUMOR PREVENTION AND TREATMENT**

(71) Applicant: Institute of Basic Medical Sciences, Chinese Academy of Medical Sciences, Beijing 100730 (CN)
(72) Inventor: CAO, Xuetao, Beijing 100730 (CN)
(74) Representative: Vossius & Partner Patentanwälte Rechtsanwälte mbB
(86) International application number: PCT/CN2023/143691
(87) International publication number: WO 2025/138291

(57) **Abstract**

The present disclosure relates to a heterogeneous nuclear ribosomal protein A2B1 (hnRNPA2B1) hnRNPA2B1 agonist compound and an anti-tumor use thereof. Specifically, the present disclosure relates to a condensed cyclic compound as represented by formula (I), a cis-trans isomer thereof, an enantiomer thereof, a diastereoisomer thereof, a racemate thereof, a solvate thereof, a hydrate or a pharmaceutically acceptable salt thereof or a prodrug thereof, a composition and product comprising any one of the foregoing, and a use of the described substances as an hnRNPA2B1 agonist in the preparation of a product for tumor prevention and/or treatment. The present disclosure can be used for broad-spectrum tumor prevention and treatment, and has a wide application prospect.

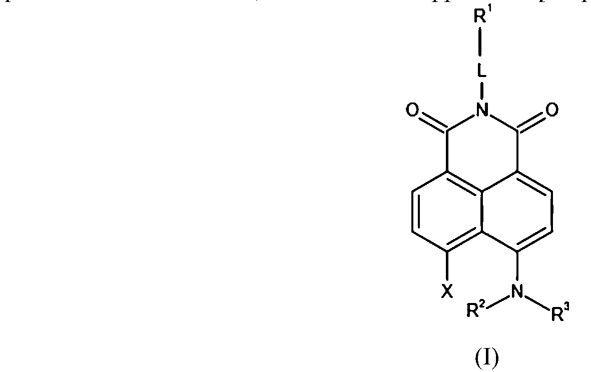

## Description

### Technical Field

The present disclosure relates to the fields of medicine and biotechnology. Specifically, the present disclosure relates to a class of compounds that serve as agonists of the ribonucleoprotein hnRNPA2B1 (heterogeneous nuclear ribonucleoprotein A2B1), products comprising said compounds, and their effects, mechanisms of action, methods of implementation, and uses in the prevention or treatment of tumor-related diseases or conditions, as well as tumor-induced damage.

### Background Art

Type I interferons are crucial for the function regulation and activation of various immune cells and are key cytokines in modulating tumor immunity. Interferons can directly inhibit the proliferation of human tumor cells and can act synergistically with various chemotherapeutic agents. Type I interferons can significantly enhance anti-tumor immune responses by inducing and activating adaptive and innate immune cells, particularly by enhancing antigen uptake, processing, presentation, and cross-presentation to T cells by dendritic cells and macrophages, thereby enhancing T cell activation. Additionally, interferons can inhibit tumor invasion by regulating the expression of proteins involved in tissue remodeling. Therefore, interferons and compounds that induce interferon production have significant utility in tumor therapy.

Defects in the interferon activation pathway within tumor cells often lead to tumor immune evasion and the failure of immune checkpoint blockade therapy. Various small-molecule drugs targeting tumors, including DNA intercalating agents, various epigenetic therapeutic drugs, and spliceosome modulators, exert their effects by inducing an increase in free self-nucleic acids, thereby initiating interferon. Recent studies have shown that the cytoplasmic cGAS-STING pathway induces Type I interferons, making the development of STING agonists an important direction in the field of tumor therapy. However, increasing evidences indicate that the cGAS-STING pathway is either poorly expressed or its signaling pathway activation is hindered in tumor cells. Furthermore, evidences also suggest that cGAS can promote tumor cell survival. Therefore, there is an urgent need in the field of tumor immunotherapy to develop agonists targeting natural immune receptors capable of inducing Type I interferons.

Heterogeneous nuclear ribonucleoprotein A2B1 (hnRNP-A2B1) is a newly identified nuclear DNA recognition receptor in recent years, belonging to the hnRNP family. On the one hand, hnRNP-A2B1 can sense and recognize nucleic acid components of DNA viruses (such as HSV-1, etc.), undergo autoactivation to form homodimers, undergo demethylation mediated by the demethylase JMJD6, translocate from the nucleus to the cytoplasm, thereby activating the TBK1-IRF3 signaling pathway and initiating Type I interferon expression to exert antiviral effects. On the other hand, as an RNA-binding protein, hnRNPA2B1 can also promote m6A modification, nucleocytoplasmic transport, and translation of cGAS, IFI16, and STING mRNAs, thereby ensuring the full induction and expression of Type I interferons.

hnRNPA2B1 is overexpressed in various tumors, including lung cancer, liver cancer, breast cancer, pancreatic cancer, glioblastoma, etc. and is generally considered an important factor for promoting tumor occurrence and development. For decades, numerous studies have shown that hnRNPA2B1 is involved in regulating various fundamental biological functions, such as regulating cell metabolism, migration and invasion, proliferation, and responses to mitochondrial stress. For example, the hnRNPA2-mediated invasive phenotype activation involves different mechanisms, including alternative splicing of TP53INP2, activation of the CXCL12/CXCR4 axis, and activation of invasive behavior following mitochondrial DNA depletion, wherein hnRNPA2 mediates this process as a novel transcriptional coactivator through interactions with NF-κB, NFAT, CREB, and C/EBPδ. Overall, hnRNPA2B1 is widely involved in regulating cancer cell phenotypes such as metabolism, proliferation, apoptosis, migration, and invasion through various molecular mechanisms. Additionally, hnRNPA2B1 has been reported to induce Epithelial-Mesenchymal Transition (EMT) in several cancer cell lines. Furthermore, hnRNPA2B1 is also involved in regulating fundamental cancer processes such as aerobic glycolysis. It has been reported that hnRNPA2 regulates the alternative splicing of pyruvate kinase isoenzyme M2 (PKM2), activating the metabolic switch of cancer cells towards aerobic glycolysis. hnRNPA2B1 also plays an important role in the hypoxia regulation. In summary, hnRNPA2B1 is widely involved in the occurrence and development of various tumors. Developing small molecules targeting hnRNPA2B1 holds promise for blocking the tumor-promoting effects of hnRNPAB1, thereby effectively aiding tumor therapy.

In conclusion, the development of specific agonists targeting hnRNPA2B1 has great potential in treating tumors. There is an urgent need in this field to develop an immunologically active substance that can initiate or promote interferon production, enhance anti-tumor effects, and effectively prevent tumor metastasis.

### Contents of the Invention

The present disclosure provides a fused cyclic compound having a structure represented by Formula (I), related derivatives thereof (e.g., cis-trans isomers, enantiomers, diastereomers, racemates, solvates, hydrates, or pharmaceutically acceptable salts or prodrugs thereof), and products comprising said compound or related derivatives. The present disclosure also provides the use of said compounds, derivatives, and products in the prevention and treatment of tumors, and further provides their use in the treatment or prevention of tumors and related diseases or conditions. The compounds, drugs, pharmaceutical compositions, or kits of the present disclosure can be used to effectively fight against tumors, control tumor occurrence and/or metastasis.

In some aspects of the present disclosure, there is provided a fused cyclic compound of Formula (I), a cis-trans isomer, an enantiomer, a diastereomer, a racemate, a solvate, a hydrate, or a pharmaceutically acceptable salt or a prodrug thereof, 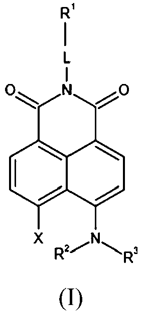 wherein:
L is -(CH₂)n-, and n is an integer from 0 to 6;
X is halogen;
R₁ is -NR⁴R⁵, wherein R⁴ and R⁵ are each independently H or C₁₋₆ alkyl;
R² and R³ are each independently H or C₁₋₆ alkyl;
or R² and R³, together with the N atom to which they are attached, form a 5- to 8-membered heterocycloalkyl group containing 1 to 3 heteroatoms selected from nitrogen, oxygen, or sulfur;
said C₁₋₆ alkyl and said 5- to 8-membered heterocycloalkyl group are unsubstituted or substituted with one or more substituents selected from the group consisting of: hydroxy, amino, halogen, C₁₋₆ alkyl, C₂₋₆ alkenyl, C₂₋₆ alkynyl, C₁₋₆ alkoxy, hydroxy C₁₋₆ alkyl, C₁₋₆ alkylamino, C₃₋₆ cycloalkyl, C₃₋₆ cycloalkyl C₁₋₆ alkyl, 3- to 6-membered heterocycloalkyl, 3- to 6-membered heterocycloalkyl C₁₋₆ alkyl, 6- to 10-membered aryl, 6- to 10-membered aryl C₁₋₆ alkyl, or 5- to 10-membered heteroaryl, and 5- to 10-membered heteroaryl C₁₋₆ alkyl.

In some aspects of the present disclosure, there is provided a use of the compound of the present disclosure in the preparation of a product for preventing and/or treating tumors.

In some aspects of the present disclosure, there is also provided a method for preventing and/or treating tumors, said method comprising administering a prophylactically and/or therapeutically effective amount of the compound or product of the present disclosure to a subject in need thereof.

In some aspects of the present disclosure, there is also provided a compound or product of the present disclosure for use in preventing and/or treating tumors.

In some aspects of the present disclosure, there is provided a use of the compound or product of the present disclosure in increasing the level of interferons (e.g., Type I interferons, such as IFN-α and/or IFN-β).

A person skilled in the art can arbitrarily combine the foregoing technical solutions and technical features without departing from the inventive concept and scope of protection of the present disclosure. Other aspects of the present disclosure will be apparent to those skilled in the art from the disclosure herein.

### Description of Drawings

The present disclosure is further described below with reference to the accompanying drawings, which are only for illustrating embodiments of the present disclosure and are not intended to limit the scope of the present disclosure.
**Figure 1****:** Activation effect of test compounds on the nuclear export of hnRNPA2B1 in A549 lung cancer cells.
**Figure 2****:** Activation effect of test compounds on downstream TBK1 and IRF3 following nuclear export of hnRNPA2B1 in A549 lung cancer cells.
**Figure 3****:** Activation effect of test compounds on Type I interferons in tumor cells.
**Figure 4****:** Inhibitory effect of test compounds on tumor cells.
**Figure 5****:** Killing effect of test compound E on tumor cells.
**Figure 6****:** Inhibitory effect of test compounds on tumor growth.
**Figure 7****:** Prolonged survival of tumor-bearing mice by test compounds.
**Figures 8-9****:** Inhibitory effect of test compounds on experimental lung metastasis of B16F10 tumors.

### Detailed Description

Through extensive research, development, and experimentation, the present application has discovered a novel class of fused cyclic compounds that can bind specifically to the hnRNPA2B1 protein with high affinity and can further extensively induce the production of higher levels of IFN-β, thereby defining them as hnRNPA2B1 agonists.

In anti-tumor tests on different types of tumor cells, the hnRNPA2B1 agonist compounds of the present disclosure can significantly activate Type I interferons in tumor cells, inhibit LLC tumor proliferation, and promote the survival of tumor-bearing mice. Furthermore, since the hnRNPA2B1 agonist compounds of the present disclosure can extensively induce the production of higher levels of IFN-β, it is anticipated that these compounds can exert anti-tumor effects through the action of Type I interferons.

Thus, the present disclosure provides methods and strategies for applying the novel anti-tumor hnRNPA2B1 agonist compounds in the prevention and treatment of tumors.

All numerical ranges provided herein are expressly intended to include all numerical values between and including the range endpoints, as well as all numerical ranges therebetween. Features mentioned in the present disclosure or features mentioned in the examples can be combined. All features disclosed in this specification can be used in combination with any composition form, and each feature disclosed in the specification can be replaced by any alternative feature that provides the same, equal, or similar purpose. Therefore, unless otherwise specified, the features disclosed are merely general examples of equivalent or similar features.

As used herein, "comprising", "having", or "including" includes "comprising", "consisting essentially of", "consisting substantially of", and "consisting of"; "consisting essentially of", "consisting substantially of", and "consisting of" are subordinate concepts of "comprising," "having," or "including."

### hnRNPA2B1 Agonist Compounds

As used herein, the terms "hnRNPA2B1 protein (polypeptide)" and "hnRNPA2B1" are used interchangeably and refer to heterogeneous nuclear ribonucleoprotein A2B1. The hnRNPA2B1 protein encompassed by the present disclosure may be a protein encoded by the *hnRNPA2B1* gene, its cDNA or CDS from an animal (e.g., human or mouse), or homologous sequences (e.g., homologous sequences of hnRNPA2B1 obtained through databases or alignment software known in the art), variants, or modified forms of these proteins that have the effect of promoting interferon expression.

As used herein, the terms "*hnRNPA2B1* gene", "hnRNPA2B1 coding gene", "hnRNPA2B1 protein-coding gene", or "nucleic acid molecule encoding hnRNPA2B1" are used interchangeably and each refers to a nucleotide sequence encoding the hnRNPA2B1 protein or polypeptide described in the present disclosure, which may be, for example, the human *hnRNPA2B1* gene with Gene ID: 3181, or the murine *hnRNPA2B1* gene with Gene ID: 53379. The term also includes molecules that hybridize to the aforementioned nucleic acid molecules under stringent conditions, or family gene molecules highly homologous to the above molecules, the expression of which is believed to be able to promote the production and effects of interferons.

The present disclosure provides a class of novel compounds that act as "agonists" (also referred to as "enhancers") of hnRNPA2B1. The terms "agonist" or "hnRNPA2B1 agonist compound" are used interchangeably and refer to a class of novel fused cyclic compounds that can increase the level or activity of hnRNPA2B1, specifically bind to the hnRNPA2B1 protein with high affinity, and further extensively induce the production of higher levels of IFN-β, thereby exerting hnRNPA2B1 agonistic effects.

The hnRNPA2B1 agonist compounds of the present disclosure can inhibit tumors, and thus can be further used for preventing or treating tumor-related or -associated diseases and/or related or associated symptoms induced by tumors.

The present application provides a fused cyclic compound represented by Formula (I), a cis-trans isomer, an enantiomer, a diastereomer, a racemate, a solvate, a hydrate, or a pharmaceutically acceptable salt or a prodrug thereof, 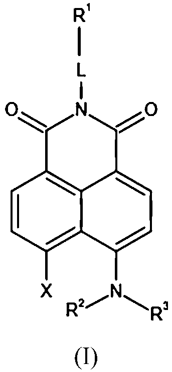 wherein:
L is -(CH₂)n-, and n is an integer from 0 to 6;
X is halogen;
R¹ is -NR⁴R⁵, wherein R⁴ and R⁵ are each independently H or C₁₋₆ alkyl;
R² and R³ are each independently H or C₁₋₆ alkyl;
or R² and R³, together with the N atom to which they are attached, form a 5- to 8-membered heterocycloalkyl group containing 1 to 3 heteroatoms selected from nitrogen, oxygen, or sulfur;
said C₁₋₆ alkyl and said 5- to 8-membered heterocycloalkyl group are unsubstituted or substituted with one or more substituents selected from the group consisting of: hydroxy, amino, halogen, C₁₋₆ alkyl, C₂₋₆ alkenyl, C₂₋₆ alkynyl, C₁₋₆ alkoxy, hydroxy C₁₋₆ alkyl, C₁₋₆ alkylamino, C₃₋₆ cycloalkyl, C₃₋₆ cycloalkyl C₁₋₆ alkyl, 3- to 6-membered heterocycloalkyl, 3- to 6-membered heterocycloalkyl C₁₋₆ alkyl, 6- to 10-membered aryl, 6- to 10-membered aryl C₁₋₆ alkyl, or 5- to 10-membered heteroaryl, and 5- to 10-membered heteroaryl C₁₋₆ alkyl.

Preferably, X is F or Cl, and more preferably, X is F.

Preferably, L is -(CH₂)n-, wherein n is 1, 2 or 3, and more preferably, n is 2.

Preferably, R⁴ and R⁵ are each independently H, methyl, or ethyl.

Preferably, R² and R³ are each independently C₁₋₃ alkyl, and preferably methyl, ethyl or isopropyl, wherein said C₁₋₃ alkyl is unsubstituted or substituted with a 3- to 8-membered heterocycloalkyl group containing 1 to 2 heteroatoms selected from nitrogen, oxygen, or sulfur; and more preferably, R² and R³ are each independently C₁₋₃ alkyl, particularly preferably methyl, ethyl, or isopropyl, wherein said C₁₋₃ alkyl is unsubstituted or substituted with a 4- to 6-membered heterocycloalkyl group containing one nitrogen heteroatom; most preferably, R² and R³ are each independently C₁₋₃ alkyl, particularly preferably methyl, ethyl, or isopropyl, wherein said C₁₋₃ alkyl is unsubstituted or substituted with a pyrrolidinyl group.

Preferably, R² and R³, together with the N atom to which they are attached, form a 5-to 8-membered heterocycloalkyl group containing two nitrogen atoms, wherein said 5- to 8-membered heterocycloalkyl group is substituted with a 3- to 8-membered heterocycloalkyl C₁₋₆ alkyl group or a hydroxy C₁₋₆ alkyl group; more preferably, R² and R³, together with the N atom to which they are attached, form a 6-membered heterocycloalkyl group containing two nitrogen atoms, wherein said 6-membered heterocycloalkyl group is substituted with a 4- to 6-membered heterocycloalkyl C₁₋₆ alkyl group or a hydroxy C₁₋₆ alkyl group; further preferably, R² and R³, together with the N atom to which they are attached, form a piperazinyl group, wherein said piperazinyl group is substituted with a 4-to 6-membered heterocycloalkyl C₁₋₆ alkyl group or a hydroxy C₁₋₆ alkyl group; most preferably, R² and R³, together with the N atom to which they are attached, form a piperazinyl group, wherein said piperazinyl group is substituted with a 5- to 6-membered heterocycloalkyl C₁₋₆ alkyl group containing one N atom or substituted with a hydroxy C₁₋₆ alkyl group, preferably, said piperazinyl group is substituted with a pyrrolidinyl C₁₋₆ alkyl group or substituted with a hydroxy C₁₋₆ alkyl group, more preferably, said piperazinyl group is substituted with a pyrrolidinylethyl group or substituted with a hydroxyethyl group.

Preferably, the compound has the following Formula (II): 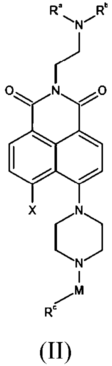
wherein, R^{a} and R^{b} are each independently H or C₁₋₆ alkyl; preferably, R^{a} and R^{b} are each independently H, methyl, or ethyl;
M is -(CH₂)n-, wherein n is 1, 2, or 3; more preferably, n is 2;
X is F or Cl; more preferably, X is F;
R^{c} is hydroxy, amino, or a 3- to 8-membered heterocycloalkyl group containing 1 to 2 heteroatoms selected from nitrogen, oxygen, or sulfur; more preferably, R^{c} is hydroxy or a 4- to 6-membered heterocycloalkyl group containing one nitrogen heteroatom; even more preferably, R^{c} is hydroxy or pyrrolidinyl; most preferably, R^{c} is hydroxy or 1-pyrrolidinyl.

Preferably, the compound is selected from the following compounds or salts thereof (e.g., hydrochloride salts):

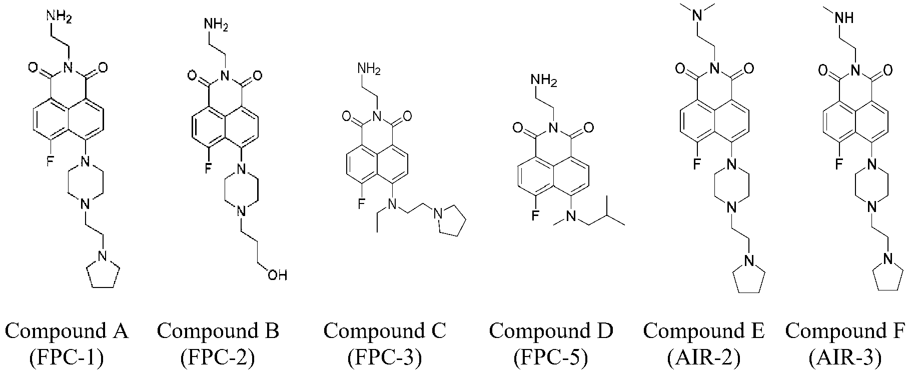

Unless otherwise defined, the following terms and phrases used herein are intended to have the following meanings. A specific term or phrase shall not be considered uncertain or unclear without a specific definition, but shall be understood according to its ordinary meaning. When a trade name appears herein, it is intended to refer to its corresponding commercial product or active ingredient.

The term "pharmaceutically acceptable" as used herein refers to those compounds, materials, compositions, and/or dosage forms that are, within the scope of sound medical judgment, suitable for use in contact with human and animal tissues without excessive toxicity, irritation, allergic response, or other problems or complications, and commensurate with a reasonable benefit/risk ratio.

The term "pharmaceutically acceptable salt(s)" refers to salt(s) of the compound(s) of the present disclosure, prepared from the compound(s) with specific substituents discovered in the present disclosure and relatively non-toxic acids or bases. When the compounds of the present disclosure contain relatively acidic functional groups, base addition salts can be obtained by contacting the neutral form of such compounds with a sufficient amount of a base, either neat or in a suitable inert solvent. Pharmaceutically acceptable base addition salts include sodium, potassium, calcium, ammonium, organic amine, or magnesium salts, or similar salts. When the compounds of the present disclosure contain relatively basic functional groups, acid addition salts can be obtained by contacting the neutral form of such compounds with a sufficient amount of an acid, either neat or in a suitable inert solvent. Examples of pharmaceutically acceptable acid addition salts include salts of inorganic acids such as hydrochloric acid, hydrobromic acid, nitric acid, carbonic acid, bicarbonate, phosphoric acid, monohydrogen phosphate, dihydrogen phosphate, sulfuric acid, bisulfate, hydroiodic acid, phosphorous acid, and the like, as well as salts of organic acids such as acetic acid, propionic acid, isobutyric acid, maleic acid, malonic acid, benzoic acid, succinic acid, suberic acid, fumaric acid, lactic acid, mandelic acid, phthalic acid, benzenesulfonic acid, *p*-toluenesulfonic acid, citric acid, tartaric acid, methanesulfonic acid, and similar acids; also include salts of amino acids (such as arginine, etc.), and salts of organic acids such as glucuronic acid. Certain specific compounds of the present disclosure contain both basic and acidic functional groups and can therefore be converted to either base or acid addition salts.

The pharmaceutically acceptable salts of the present disclosure can be synthesized from the parent compound containing an acid group or a base group by conventional chemical methods. Generally, such salts are prepared by reacting these compounds in free acid or base forms with a stoichiometric amount of the appropriate base or acid, respectively, in water or an organic solvent, or a mixture of both.

The compounds of the present disclosure may exist in specific geometric isomers or stereoisomeric forms. The present invention contemplates all such compounds, including *cis*- and *trans*-isomers, *(-)*- and *(+)*-enantiomers, (*R*)- and (*S*)-enantiomers, diastereomers, (*D*)-isomers, (*L*)-isomers, and racemic mixtures and other mixtures thereof, for example, enantiomerically or diastereomerically enriched mixtures, all of which fall within the scope of the present disclosure. Additional asymmetric carbon atoms may be present in substituents, such as alkyl groups. All such isomers, as well as mixtures thereof, are included within the scope of the present disclosure.

Unless otherwise specified, the term "enantiomer" or "optical isomer" refers to stereoisomers that are mirror images of one another.

Unless otherwise specified, the term "cis-trans isomer" or "geometric isomer" arises from the inability of double bonds or single bonds of ring-forming carbon atoms to rotate freely.

Unless otherwise specified, the term "diastereomer" refers to stereoisomers whose molecules have two or more chiral centers and are non-mirror images of one another.

The compounds of the present disclosure may exist in specific conformations. Unless otherwise specified, the term "tautomer" or "tautomeric form" refers to different functional group isomers that are in dynamic equilibrium at room temperature and can rapidly interconvert. If tautomers are possible (e.g., in solution), a chemical equilibrium of tautomers may be achieved. For example, proton tautomers (also known as prototropic tautomers) include interconversions via proton migration, such as keto-enol tautomerism and imine-enamine tautomerism. Valence tautomers include interconversions via the reorganization of some bonding electrons. A specific example of keto-enol tautomerism is the interconversion between the two tautomers pentane-2,4-dione and 4-hydroxy-3-en-2-one.

The term "prodrug" generally refers to a derivative produced by derivatizing the functional groups in the compound of Formula (I), which can be readily converted back into the compound of Formula (I) *in vivo*. Suitable prodrug selection and preparation can generally be found, for example, in Design of Prodrugs, ed. H. Bundgaard, Elsevier, 1985.

The compounds of the present disclosure may contain unnatural proportions of atomic isotopes at one or more of the atoms that constitute the compound. Said isotopes have the same atomic number, but an atomic mass or mass number different from the atomic mass or mass number predominantly found in nature. For example, the compound may be labeled with radioactive isotopes, such as deuterium (²H), tritium (³H), iodine-125 (¹²⁵I), or carbon-14 (¹⁴C). All isotopic variations of the compounds of the present disclosure, whether radioactive or not, are included within the scope of the present disclosure. Isotopic variants may offer certain therapeutic advantages. For example, substitution of hydrogen with deuterium can form deuterated drugs. The bond between deuterium and carbon is stronger than that between ordinary hydrogen and carbon. Compared to non-deuterated drugs, deuterated drugs may offer advantages, such as reduced toxic side effects, increased drug stability, enhanced efficacy, and prolonged drug biological half-life, or they may provide standard compounds useful for the characterization of biological samples. Isotopically enriched compounds within Formula (I) can be prepared without undue experimentation by conventional techniques well known to those skilled in the art, or by methods analogous to those described in the schemes and examples herein, using appropriate isotopically enriched reagents and/or intermediates.

"Optional" or "optionally" means that the subsequently described event or circumstance may, but need not, occur, and that the description includes instances where said event or circumstance occurs and instances where it does not.

The naming rules used in the present disclosure are based on the IUPAC systematic nomenclature generated by ChemDraw software. Any open valence bond appearing on a carbon, oxygen, sulfur, or nitrogen atom in the structures presented in the present disclosure indicates the presence of a hydrogen atom

The term "substituted" means that any one or more hydrogen atoms on a specified atom are replaced by a substituent, which may include deuterium and hydrogen variants, provided that the valence of the specified atom is normal and the substituted compound is stable. When the substituent is oxo (i.e., =O), it means that two hydrogen atoms are replaced; oxo substitution does not occur on aromatic groups. The term "optionally substituted" means that it may be substituted or unsubstituted. Unless otherwise specified, the type and number of substituents can be arbitrary as long as they are chemically feasible.

When any variable (e.g., R) appears more than once in the composition or structure of a compound, its definition at each occurrence is independent. Thus, for example, if a group is substituted with 0-2 R groups, then said group may optionally be substituted with up to two R groups, and R has an independent option at each occurrence. Furthermore, combinations of substituents and/or their variants are permissible only if such combinations result in stable compounds.

When the number of a linking group is zero, e.g., -(CRR)o-, it means the linking group is a single bond.

When a variable is selected as a single bond, it indicates that the two groups it connects are directly linked. For example, when L in A-L-Z represents a single bond, the structure is actually A-Z.

When the direction of attachment of a listed linking group is not specified, the attachment direction is arbitrary. For example, if the linking group L in 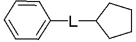 is 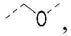 then 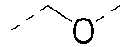 can link the benzene ring and the cyclopentyl group in the same direction as the left-to-right reading order to form 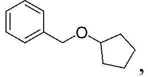 or in the opposite direction to the left-to-right reading order to form 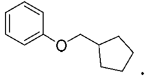 Combinations of the linking groups, substituents and/or their variants are permissible only if such combinations result in stable compounds.

Unless otherwise specified, the number of atoms in a ring is generally defined as the ring size. For example, a "3- to 7-membered ring" refers to a ring in which 3 to 7 atoms are arranged in a ring.

Unless otherwise specified, the term "halogen" refers to fluorine, chlorine, bromine, and iodine.

Unless otherwise specified, the term "C₁₋₆ alkyl" is used to denote a straight-chain or branched saturated hydrocarbon group consisting of 1 to 6 carbon atoms. Said C₁₋₆ alkyl includes C₁₋₅, C₁₋₄, C₁₋₃, C₁₋₂, C₂₋₆, C₂₋₄, C₆, C₅ alkyl, etc.; it may be monovalent (e.g., methyl), divalent (e.g., methylene), or multivalent (e.g., methine). Examples of C₁₋₆ alkyl include, but are not limited to, methyl (Me), ethyl (Et), propyl (including n-propyl and isopropyl), butyl (including *n*-butyl, isobutyl, *s*-butyl, and *t*-butyl), pentyl (including *n-*pentyl, isopentyl, and neopentyl), hexyl, etc.

Unless otherwise specified, the term "C₁₋₃ alkyl" is used to denote a straight-chain or branched saturated hydrocarbon group consisting of 1 to 3 carbon atoms. Said C₁₋₃ alkyl includes C₁₋₂, C₂₋₃ alkyl, etc.; it may be monovalent (e.g., methyl), divalent (e.g., methylene), or multivalent (e.g., methine). Examples of C₁₋₃ alkyl include, but are not limited to, methyl (Me), ethyl (Et), propyl (including *n*-propyl and isopropyl), etc.

Unless otherwise specified, the term "C₂₋₆ alkenyl" is used to denote a straight-chain or branched hydrocarbon group consisting of 2 to 6 carbon atoms and containing at least one carbon-carbon double bond. The carbon-carbon double bond can be located at any position of the group. Said C₂₋₆ alkenyl includes C₂₋₄, C₂₋₃, C₄, C₃, C₂ alkenyl, etc.; it may be monovalent, divalent, or multivalent. Examples of C₂₋₆ alkenyl include, but are not limited to, vinyl, propenyl, butenyl, pentenyl, hexenyl, butadienyl, pentadienyl, etc.

Unless otherwise specified, the term "C₁₋₆ alkoxy" denotes those alkyl groups containing 1 to 6 carbon atoms attached to the rest of the molecule through an oxygen atom. Said C₁₋₆ alkoxy includes C₁₋₄, C₁₋₃, C₁₋₂, C₂₋₆, C₂₋₄, C₆, C₅, C₄, C₃ alkoxy, etc. Examples of C₁₋₆ alkoxy include, but are not limited to, methoxy, ethoxy, propoxy (including *n*-propoxy and isopropoxy), butoxy (including *n*-butoxy, isobutoxy, *s*-butoxy, and *t*-butoxy), pentyloxy (including *n*-pentyloxy, isopentyloxy, and neopentyloxy), hexyloxy, etc.

Unless otherwise specified, the term "C₁₋₆ alkylamino" denotes those alkyl groups containing 1 to 6 carbon atoms attached to the rest of the molecule through an amino group. Said C₁₋₆ alkylamino includes C₁₋₄, C₁₋₃, C₁₋₂, C₂₋₆, C₂₋₄, C₆, C₅, C₄, C₃, and C₂ alkylamino, etc. Examples of C₁₋₆ alkylamino include, but are not limited to, -NHCH₃, -N(CH₃)₂, - NHCH₂CH₃, -N(CH₃)CH₂CH₃, -N(CH₂CH₃)₂, -NHCH₂CH₂CH₃, -NHCH(CH₃)₂, - NHCH₂CH₂CH₂CH₃, etc.

Unless otherwise specified, the term "C₃₋₆ cycloalkyl" denotes a saturated cyclic hydrocarbon group consisting of 3 to 6 carbon atoms, which is a monocyclic or bicyclic ring system. Said C₃₋₆ cycloalkyl includes C₃₋₅, C₄₋₅, C₅₋₆ cycloalkyl, etc.; it may be monovalent, divalent, or multivalent. Examples of C₃₋₆ cycloalkyl include, but are not limited to, cyclopropyl, cyclobutyl, cyclopentyl, cyclohexyl, etc.

Unless otherwise specified, the term "3- to 8-membered heterocycloalkyl", by itself or in combination with other terms, denotes a saturated monocyclic group consisting of 3 to 8 ring atoms, of which 1, 2, 3, or 4 ring atoms are heteroatoms independently selected from O, S, and N, with the remainder being carbon atoms, wherein the nitrogen atom is optionally quaternized, and the nitrogen and sulfur heteroatoms may optionally be oxidized (i.e., NO and S(O)_{z}, where z is 1 or 2). Furthermore, for the "3- to 8-membered heterocycloalkyl", the heteroatom may occupy the attachment position of the heterocycloalkyl to the rest of the molecule. The 3- to 8-membered heterocycloalkyl includes 4- to 6-membered, 5- to 6-membered, 7- to 8-membered, 4-membered, 5-membered, 6-membered heterocycloalkyl, etc. Examples of 3- to 6-membered heterocycloalkyl include, but are not limited to, azetidinyl, oxetanyl, thietanyl, pyrrolidinyl, pyrazolidinyl, imidazolidinyl, tetrahydrothienyl, tetrahydrofuranyl (including tetrahydrofuran-2-yl), piperidinyl, tetrahydropyranyl, tetrahydrothiopyranyl, piperazinyl, morpholinyl, thiomorpholinyl, etc.

Unless otherwise specified, the terms "6- to 10-membered aromatic ring" and "6- to 10-membered aryl" are used interchangeably, and the term "6- to 10-membered aryl" denotes a monovalent aromatic carbocyclic ring system containing 6 to 10 carbon atoms and having at least one aromatic ring or a fused polycyclic system in which at least one ring is aromatic. Examples of aryl groups include, but are not limited to, phenyl, naphthyl, biphenyl, indanyl, and the like.

Unless otherwise specified, the terms "5- to 10-membered heteroaromatic ring" and "5- to 10-membered heteroaryl" are used interchangeably, and the term "5- to 10-membered heteroaryl" denotes a cyclic group having a conjugated π-electron system consisting of 5 to 10 ring atoms, of which 1, 2, 3, or 4 ring atoms are heteroatoms independently selected from O, S, and N, with the remainder being carbon atoms. It can be a monocyclic or fused bicyclic ring system, wherein at least one ring in the system is aromatic. The nitrogen atom is optionally quaternized, and the nitrogen and sulfur heteroatoms may optionally be oxidized (i.e., NO and S(O)_{z}, where z is 1 or 2). The 5- to 10-membered heteroaryl group can be attached to the rest of the molecule via a heteroatom or a carbon atom. The 5- to 10-membered heteroaryl includes 5- to 8-membered, 5- to 7-membered, 5- to 6-membered, 5-membered, 6-membered heteroaryl, and the like. Examples of the 5- to 10-membered heteroaryl include, but are not limited to, pyrrolyl (including N-pyrrolyl, 2-pyrrolyl, 3-pyrrolyl, etc.), pyrazolyl (including 2-pyrazolyl, 3-pyrazolyl, etc.), imidazolyl (including N-imidazolyl, 2-imidazolyl, 4-imidazolyl, 5-imidazolyl, etc.), oxazolyl (including 2-oxazolyl, 4-oxazolyl, 5-oxazolyl, etc.), triazolyl (1*H*-1,2,3-triazolyl, 2*H*-1,2,3-triazolyl, *1H-1,2,4-*triazolyl, and 4*H*-1,2,4-triazolyl), tetrazolyl, isoxazolyl (including 3-isoxazolyl, 4-isoxazolyl, 5-isoxazolyl, etc.), thiazolyl (including 2-thiazolyl, 4-thiazolyl, 5-thiazolyl, etc.), furanyl (including 2-furanyl, 3-furanyl, etc.), thienyl (including 2-thienyl, 3-thienyl, etc.), pyridinyl (including 2-pyridinyl, 3-pyridinyl, 4-pyridinyl, etc.), pyrazinyl, pyrimidinyl (including 2-pyrimidinyl, 4-pyrimidinyl, etc.), benzothiazolyl (including 2-benzothiazolyl, etc.), purinyl, benzimidazolyl (including 2-benzimidazolyl, etc.), benzoxazolyl, indazolyl (including 5-indazolyl, etc.), isoquinolinyl (including 1-isoquinolinyl, 5-isoquinolinyl, etc.), quinoxalinyl (including 2-quinoxalinyl, 5-quinoxalinyl, etc.), or quinolinyl (including 3-quinolinyl, 6-quinolinyl, etc.), 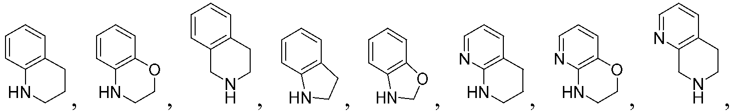 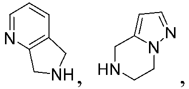 and the like.

Unless otherwise specified, the terms "5- to 6-membered heteroaromatic ring" and "5-to 6-membered heteroaryl" are used interchangeably, and the term "5- to 6-membered heteroaryl" denotes a cyclic group having a conjugated π-electron system consisting of 5 to 6 ring atoms, of which 1, 2, 3, or 4 ring atoms are heteroatoms independently selected from O, S, and N, with the remainder being carbon atoms. The nitrogen atom is optionally quaternized, and the nitrogen and sulfur heteroatoms may optionally be oxidized (i.e., NO and S(O)_{z}, where z is 1 or 2). The 5- to 6-membered heteroaryl group can be attached to the rest of the molecule via a heteroatom or a carbon atom. The 5- to 6-membered heteroaryl includes 5-membered and 6-membered heteroaryl, and the like. Examples of the 5- to 6-membered heteroaryl include, but are not limited to, pyrrolyl (including *N*-pyrrolyl, 2-pyrrolyl, 3-pyrrolyl, etc.), pyrazolyl (including 2-pyrazolyl, 3-pyrazolyl, etc.), imidazolyl (including *N*-imidazolyl, 2-imidazolyl, 4-imidazolyl, 5-imidazolyl, etc.), oxazolyl (including 2-oxazolyl, 4-oxazolyl, 5-oxazolyl, etc.), triazolyl (1*H*-1,2,3-triazolyl, 2*H*-1,2,3-triazolyl, 1*H*-1,2,4-triazolyl, and 4*H*-1,2,4-triazolyl), tetrazolyl, isoxazolyl (including 3-isoxazolyl, 4-isoxazolyl, 5-isoxazolyl, etc.), thiazolyl (including 2-thiazolyl, 4-thiazolyl, 5-thiazolyl, etc.), furanyl (including 2-furanyl, 3-furanyl, etc.), thienyl (including 2-thienyl, 3-thienyl, etc.), pyridinyl (including 2-pyridinyl, 3-pyridinyl, 4-pyridinyl, etc.), pyrazinyl, pyrimidinyl (including 2-pyrimidinyl, 4-pyrimidinyl, etc.), and the like.

Unless otherwise specified, Cₙ₋ₙ₊ₘ or Cₙ-Cₙ₊ₘ includes any specific case of n to n+m carbon atoms, for example, C₁₋₇ includes C₁, C₂, C₃, C₄, C₅, C₆, and C₇, and also includes any range within n to n+m, for example, C₁₋₇ includes C₁₋₃, C₁₋₆, C₃₋₆, C₄₋₇, C₅₋₇, etc. Similarly, "n-membered to n+m-membered" means the number of ring atoms is n to n+m, for example, a 3- to 7-membered ring includes 3-membered, 4-membered, 5-membered, 6-membered, and 7-membered rings, and also includes any range within n to n+m, for example, a 3- to 7-membered ring includes 3- to 6-membered, 4- to 7-membered, 5- to 7-membered, 6- to 7-membered rings, etc.

### Preparation Methods

The present disclosure also relates to methods for producing the compounds of Formula (I) as defined above. In some embodiments, the synthetic methods for the compounds of the present disclosure are shown in the scheme below:

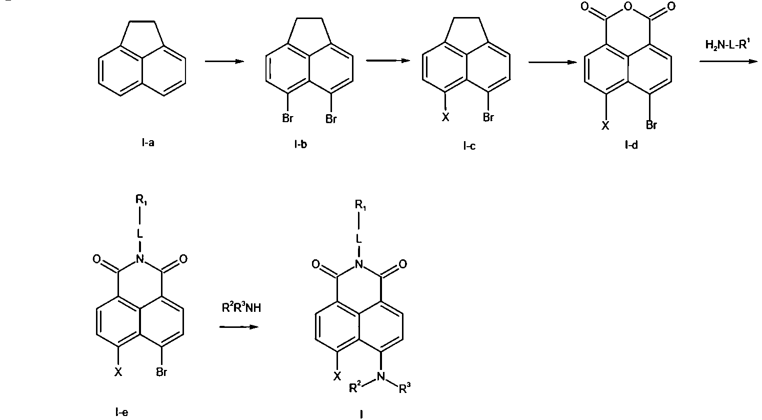

When X is selected from halogens other than Br, such as F, Cl, I, etc., the method comprises the following steps:
a) brominating the compound of Formula I-a using a brominating agent to produce the compound of Formula I-b. Preferably, the brominating agent includes, but is not limited to, N-bromosuccinimide (NBS) or 1,3-dibromo-5,5-dimethylhydantoin. Preferably, the reaction temperature is 0-30 °C;
b) replacing one Br atom in the compound of Formula I-b with other halogen atom to obtain the compound of Formula I-c. Preferably, step b) is carried out using 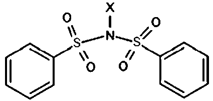 and an organometallic salt strong base at low temperature, wherein X is a halogen other than Br, such as F, Cl, or I. More preferably, the organometallic salt strong base is n-butyllithium. Preferably, the reaction temperature is -78 °C;
c) oxidizing the compound of Formula I-c using an oxidizing agent to produce the compound of Formula I-d. Preferably, the oxidizing agent includes, but is not limited to, K₂Cr₂O₇. Preferably, the reaction temperature is 60-150 °C;
d) reacting the compound of Formula I-d with H₂N-L-R¹ to obtain the compound of Formula I-e. Preferably, the reaction temperature is 50-150 °C;
e) reacting the compound of Formula I-e with R²R³NH to obtain the compound of Formula I. Preferably, the reaction is carried out in the presence of a catalyst, bis(triphenylphosphine)palladium(II) chloride. Preferably, the reaction temperature is 20-150 °C.

When X is Br, the method comprises:
a) brominating the compound of Formula I-a using a brominating agent to produce the compound of Formula I-b. Preferably, the brominating agent includes, but is not limited to, N-bromosuccinimide (NBS). Preferably, the reaction temperature is 0-30 °C;
c) oxidizing the compound of Formula I-b using an oxidizing agent to produce the compound of Formula I-d. Preferably, the oxidizing agent includes, but is not limited to, K₂Cr₂O₇. Preferably, the reaction temperature is 60-150 °C;
d) reacting the compound of Formula I-d with H₂N-L-R¹ to obtain the compound of Formula I-e. Preferably, the reaction temperature is 50-150 °C;
e) reacting the compound of Formula I-e with R²R³NH to obtain the compound of Formula I. Preferably, the reaction is carried out in the presence of a catalyst, bis(triphenylphosphine)palladium(II) chloride. Preferably, the reaction temperature is 20-150 °C.

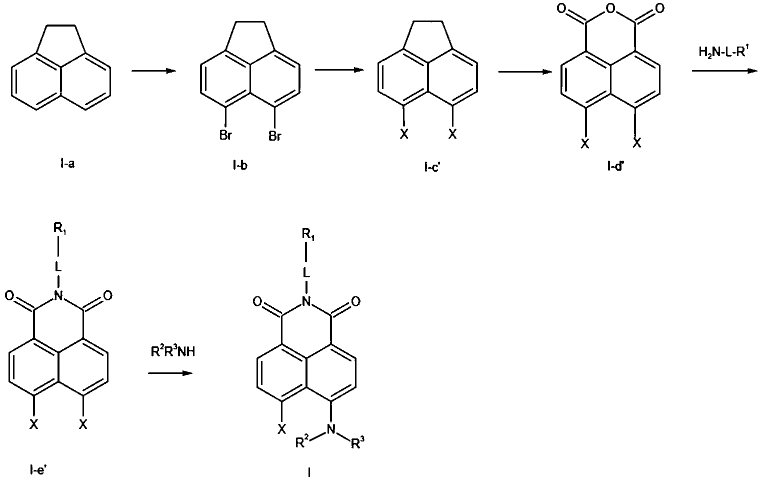

Alternatively, when X is selected from halogens other than Br, such as F, Cl, I, etc., the method comprises the following steps:
f) brominating the compound of Formula I-a using a brominating agent to produce the compound of Formula I-b. Preferably, the brominating agent includes, but is not limited to, N-bromosuccinimide (NBS) or 1,3-dibromo-5,5-dimethylhydantoin. Preferably, the reaction temperature is 0-30 °C;
g) replacing both Br atoms in the compound of Formula I-b with other halogen atoms to obtain the compound of Formula I-c'. Preferably, step (g) is carried out using 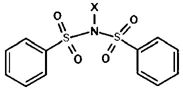 and an organometallic salt strong base at low temperature, wherein X is a halogen other than Br, such as F, Cl, or I. More preferably, the organometallic salt strong base is n-butyllithium. Preferably, the reaction temperature is -78 °C;
h) oxidizing the compound of Formula I-c' using an oxidizing agent to produce the compound of Formula I-d'. Preferably, the oxidizing agent includes, but is not limited to, K₂Cr₂O₇. Preferably, the reaction temperature is 60-150 °C;
i) reacting the compound of Formula I-d' with H₂N-L-R¹ to obtain the compound of Formula I-e'. Preferably, the reaction temperature is 50-150 °C;
j) reacting the compound of Formula I-e' with R²R³NH to obtain the compound of Formula I. Preferably, the reaction is carried out in the presence of a catalyst, bis(triphenylphosphine)palladium(II) chloride. Preferably, the reaction temperature is 20-150 °C.

As known to those skilled in the art, certain reactive groups (e.g., -NH₂, -OH, etc.) require routine protection and deprotection when necessary, which is familiar to those skilled in the art.

### Product and Uses Thereof

The present disclosure also provides a product, which may be, for example, a drug, a pharmaceutical composition, or a kit/a medicine kit, comprising an effective amount of the hnRNPA2B1 agonist compound of the present disclosure, and a pharmaceutically or immunologically acceptable carrier. As used herein, the terms "active substance" or "active substance of the present disclosure" are used interchangeably and refer to the hnRNPA2B1 agonist compound having the structural formula (I), or a derivative or product thereof.

As used herein, the term "pharmaceutically acceptable" component refers to a substance that is suitable for use in humans and/or animals without undue adverse reactions (such as toxicity, irritation, and allergic reactions), i.e., having a reasonable benefit/risk ratio. As used herein, the term "effective amount" refers to an amount that produces a function or activity in humans and/or animals and is acceptable to humans and/or animals.

As used herein, the term "pharmaceutically acceptable carrier" refers to a carrier for the administration of a therapeutic agent, including various excipients and diluents. The term refers to such agent carriers that are themselves not essential active ingredients and that have no excessive toxicity after administration. Suitable carriers are well known to those of ordinary skill in the art, and a full discussion of pharmaceutically acceptable excipients can be found in Remington's Pharmaceutical Sciences (Mack Pub. Co., N.J. 1991).

The pharmaceutically acceptable carrier in the composition may contain liquids, such as water, saline, glycerol, and ethanol. Additionally, auxiliary substances may be present in these carriers, such as fillers, disintegrants, lubricants, glidants, effervescent agents, wetting or emulsifying agents, flavoring agents, pH buffering substances, and the like. Generally, these substances can be formulated in a non-toxic, inert, and pharmaceutically acceptable aqueous carrier medium, wherein the pH is generally about 5-8, preferably about 6-8.

The active substance in the product of the present disclosure is present in an amount of 0.001 to 99.9 wt% based on the total weight of the composition; preferably 1 to 95 wt% based on the total weight of the composition, more preferably 5 to 90 wt%, even more preferably 10 to 80 wt%. The remainder is the pharmaceutically acceptable carrier and other additives.

As used herein, the term "unit dosage form" refers to a dosage form prepared for single administration for convenience of taking, including but not limited to various solid dosage forms (e.g., tablets), liquid dosage forms, capsules, and sustained-release dosage forms.

In another preferred embodiment of the present disclosure, the product is in a unit dosage form or a multiple dosage form, and the content of the active substance is 0.01 to 2000 mg/dosage unit, preferably 0.1 to 1500 mg/dosage unit, more preferably 1 to 1000 mg/dosage unit. In another preferred embodiment of the present disclosure, 1 to 6 doses of the composition of the present disclosure are administered per day, preferably 1 to 3 doses; most preferably, the daily dose is 1 dose.

It should be understood that the effective dosage of the active substance such as the hnRNPA2B1 agonist compound may vary with the severity of the condition to be treated or administered. The specific situation is determined according to the individual conditions of the subject (e.g., subject's weight, age, physical condition, desired effect), which is within the judgment of a skilled physician.

The product of the present disclosure can be solid (e.g., granules, tablets, lyophilized powder, suppositories, capsules, sublingual tablets) or liquid (e.g., oral liquid) or other suitable shapes. Routes of administration may include, but are not limited to: (1) conventional administration methods, such as gastrointestinal administration (e.g., oral), parenteral administration (e.g., solution injection, intravenous infusion), such as mucosal administration, transdermal administration, respiratory nebulized inhalation, nasal drops, spray, oral administration, intramuscular injection, and/or intravenous administration; (2) linking the hnRNPA2B1 agonist to a transferrin/poly-L-lysine complex to enhance its biological effects; (3) encapsulating the drug with liposomes to mediate its entry into cells, which not only facilitates the smooth entry of the compound molecule, but also protects it from hydrolysis by various extracellular enzymes; (4) using liposomes to transport the active substance to specifically transport it to target tissues and target cells.

Furthermore, the product of the present disclosure may also contain other active substance(s) for improving and treating tumors or may be combined with other tumor prevention and treatment methods. In some embodiments, before, simultaneously with, or after administration of the product of the present disclosure, other active substance(s) that regulate anti-tumor activity are administered.

In some embodiments, activation of hnRNPA2B1 by the compound or product of the present application further modulates the immune system to prevent and treat tumors.

The term "subject" can refer to an animal, including but not limited to, primates (e.g., humans), monkeys, cows, pigs, sheep, goats, horses, dogs, cats, rabbits, rats, or mice. The terms "subject" and "patient" are used interchangeably herein, for example, to refer to a mammalian subject, such as a human subject.

In the context of treating a disease, disorder, or condition, the terms "treatment," "treating," and "therapy" are intended to include alleviating or eliminating the disease, disorder, or condition or one or more symptoms associated with the disease, disorder, or condition; or slowing the progression, spread, or worsening of the disease, disorder, or condition or one or more symptoms thereof. "Cancer treatment" refers to one or more of the following effects: (1) inhibiting tumor growth to some extent, including (i) slowing and (ii) completely inhibiting the growth; (2) reducing the number of tumor cells; (3) maintaining tumor size; (4) reducing tumor size; (5) inhibiting, including (i) reducing, (ii) slowing, or (iii) completely preventing tumor cell infiltration into peripheral organs; (6) inhibiting, including (i) reducing, (ii) slowing, or (iii) completely preventing cancer metastasis; (7) enhancing an anti-tumor immune response, which may (i) maintain tumor size, (ii) reduce tumor size, (iii) slow tumor growth, (iv) reduce, slow, or prevent invasion, and/or (8) alleviating to some extent the severity or number of one or more symptoms associated with the disorder.

The compounds disclosed herein can be hnRNPA2B1 agonists. These compounds can potentially be used to treat diseases or disorders, including but not limited to, cell proliferative diseases. Cell proliferative diseases include, but are not limited to, cancer, benign papillomatosis, gestational trophoblastic disease, and benign neoplastic diseases, such as skin papillomas (warts) and genital papillomas.

In specific embodiments, the disease or disorder to be treated is a cell proliferative disease. In certain embodiments, the cell proliferative disease is cancer. In specific embodiments, the cancer is selected from: brain and spinal cancers, head and neck cancers, leukemias and hematological cancers, skin cancers, reproductive system cancers, gastrointestinal system cancers, liver and bile duct cancers, pancreatic cancers, kidney cancers, prostate cancers and bladder cancers, bone cancers, lung cancers, malignant mesothelioma, sarcomas, lymphomas, adenocarcinomas, thyroid cancers, cardiac tumors, germ cell tumors, malignant neuroendocrine (carcinoid) tumors, midline tract carcinomas, and cancers of unknown primary (i.e., cancer where metastatic cancer is found but the original cancer site is unknown). In specific embodiments, the cancer is present in an adult patient. In additional embodiments, the cancer is present in a pediatric patient. In specific embodiments, the cancer is associated with AIDS.

In specific embodiments, the cancer is selected from brain and spinal cancer. In specific embodiments, the cancer is selected from: anaplastic astrocytoma, glioblastoma, astrocytoma, and esthesioneuroblastoma (also known as olfactory neuroblastoma). In specific embodiments, the brain cancer is selected from: astrocytoma (e.g., cellular astrocytoma, subependymal giant cell astrocytoma, diffuse astrocytoma, pleomorphic xanthoastrocytoma, anaplastic astrocytoma, astrocytoma, giant cell glioblastoma, glioblastoma, secondary glioblastoma, primary adult glioblastoma, and primary pediatric glioblastoma), maxillary nerve tumor (e.g., oligodendroglioma and anaplastic oligodendroglioma), oligoastrocytic tumor (e.g., oligoastrocytoma and anaplastic oligoastrocytoma), ependymoma (e.g., myxopapillary ependymoma and anaplastic ependymoma); medulloblastoma, primitive neuroectodermal tumors, schwannoma, meningioma, atypical meningioma, anaplastic meningioma, pituitary adenoma, brainstem glioma, cerebellar astrocytoma, cerebral aneurysm/malignant glioma, optic pathway and hypothalamic glioma, and primary central nervous system lymphoma. In specific examples of these embodiments, the brain cancer is selected from: glioma, glioblastoma multiforme, paraganglioma, and supratentorial primitive neuroectodermal tumor (sPNET).

In specific embodiments, the cancer is selected from: head and neck cancers, including nasopharyngeal carcinoma, nasal cavity and paranasal sinus cancer, hypopharyngeal cancer, oral cavity cancer (e.g., squamous cell carcinoma, lymphoma, and sarcoma), lip cancer, oropharyngeal cancer, salivary gland tumor, laryngeal cancer (e.g., laryngeal squamous cell carcinoma, rhabdomyosarcoma), and eye cancer or ocular cancer. In specific embodiments, the eye cancer is selected from intraocular melanoma and retinoblastoma.

In specific embodiments, the cancer is selected from leukemia and hematological cancer. In specific embodiments, the cancer is selected from: myeloproliferative neoplasm, myelodysplastic syndrome, myelodysplastic/myeloproliferative neoplasm, acute myeloid leukemia (AML), myelodysplastic syndrome (MDS), chronic myeloid leukemia (CML), myeloproliferative neoplasm (MPN), post-MPN AML, post-MDS AML, del(5q)-associated high-risk MDS or AML, blastic phase chronic myeloid leukemia, angioimmunoblastic lymphoma, acute lymphoblastic leukemia, Langerhans cell histiocytosis, hairy cell leukemia, and plasmacytoma (including plasmacytoma and multiple myeloma). Leukemias referred to herein can be acute or chronic.

In specific embodiments, the cancer is selected from skin cancer. In specific embodiments, the skin cancer is selected from melanoma, squamous cell carcinoma, and basal cell carcinoma.

In specific embodiments, the cancer is selected from cancers of the reproductive system. In specific embodiments, the cancer is selected from: breast cancer, cervical cancer, vaginal cancer, ovarian cancer, prostate cancer, penile cancer, and testicular cancer. In specific examples of these embodiments, the cancer is breast cancer, which is selected from ductal carcinoma and phyllodes tumor. In specific examples of these embodiments, the breast cancer can be male breast cancer or female breast cancer. In specific examples of these embodiments, the cancer is cervical cancer, which is selected from squamous cell carcinoma and adenocarcinoma. In specific examples of these embodiments, the cancer is ovarian cancer, which is selected from epithelial carcinoma.

In specific embodiments, the cancer is selected from cancers of the gastrointestinal system. In specific embodiments, the cancer is selected from: esophageal cancer, gastric cancer (also known as stomach cancer), gastrointestinal carcinoid tumor, pancreatic cancer, gallbladder cancer, colorectal cancer, and anal cancer. In examples of these embodiments, the cancer is selected from: esophageal squamous cell carcinoma, esophageal adenocarcinoma, gastric adenocarcinoma, gastrointestinal carcinoid tumor, gastrointestinal stromal tumor, gastric lymphoma, gastrointestinal lymphoma, solid pseudopapillary tumor of the pancreas, pancreatoblastoma, islet cell tumor, pancreatic cancer (including acinar cell carcinoma and ductal adenocarcinoma), gallbladder adenocarcinoma, colorectal adenocarcinoma, and anal squamous cell carcinoma.

In specific embodiments, the cancer is selected from liver cancer and bile duct cancer. In specific embodiments, the cancer is liver cancer (also known as hepatocellular carcinoma). In specific embodiments, the cancer is bile duct cancer (also known as cholangiocarcinoma). In examples of these embodiments, the bile duct cancer is selected from intrahepatic cholangiocarcinoma and extrahepatic cholangiocarcinoma.

In specific embodiments, the cancer is selected from kidney cancer and bladder cancer. In specific embodiments, the cancer is kidney cancer, which is selected from: renal cell carcinoma, Wilms tumor, and transitional cell carcinoma. In specific embodiments, the cancer is bladder cancer, which is selected from: ureteral cancer (transitional cell carcinoma), squamous cell carcinoma, and adenocarcinoma.

In specific embodiments, the cancer is selected from bone cancer. In specific embodiments, the bone cancer is selected from: osteosarcoma, malignant fibrous histiocytoma of bone, Ewing's sarcoma, chordoma (bone cancer along the spine).

In specific embodiments, the cancer is selected from lung cancer. In specific embodiments, the lung cancer is selected from: non-small cell lung cancer, small cell lung cancer, bronchial tumor, and pleuropulmonary blastoma.

In specific embodiments, the cancer is selected from malignant mesothelioma. In specific embodiments, the cancer is selected from epithelial mesothelioma and sarcomatoid mesothelioma.

In specific embodiments, the cancer is selected from sarcoma. In specific embodiments, the sarcoma is selected from: central chondrosarcoma, central and periosteal chondroma, fibrosarcoma, clear cell sarcoma of the tendon sheath, and Kaposi's sarcoma.

In specific embodiments, the cancer is selected from lymphomas. In specific embodiments, the cancer is selected from: Hodgkin's lymphoma (e.g., Reed-Sternberg cells), non-Hodgkin's lymphoma (e.g., diffuse large B-cell lymphoma, follicular lymphoma, mycosis fungoides, Sezary syndrome, primary central nervous system lymphoma), cutaneous T-cell lymphoma, primary central nervous system lymphoma.

In specific embodiments, the cancer is selected from adenocarcinoma. In specific embodiments, the cancer is selected from: adrenocortical carcinoma (also known as adrenal cortical carcinoma or adrenocortical carcinoma), pheochromocytoma, paraganglioma, pituitary tumor, thymoma, and thymic carcinoma.

In specific embodiments, the cancer is selected from thyroid cancer. In specific embodiments, the thyroid cancer is selected from: medullary thyroid cancer, papillary thyroid cancer, and follicular thyroid cancer.

In specific embodiments, the cancer is selected from germ cell tumor. In specific embodiments, the cancer is selected from: malignant extracranial germ cell tumor and malignant extragonadal germ cell tumor. In specific examples of these embodiments, the malignant extragonadal germ cell tumor is selected from non-seminoma and seminoma.

In specific embodiments, the cancer is selected from cardiac tumor. In specific embodiments, the cardiac tumor is selected from: malignant teratoma, lymphoma, rhabdomyosarcoma, angiosarcoma, chondrosarcoma, infantile fibrosarcoma, and synovial sarcoma.

In specific embodiments, the cell proliferative disease is selected from: benign papillomatosis, benign neoplastic diseases, and gestational trophoblastic disease. In specific embodiments, the benign neoplastic disease is selected from: skin papilloma (warts) and genital papilloma. In specific embodiments, the gestational trophoblastic disease is selected from: hydatidiform mole and gestational trophoblastic neoplasia (e.g., invasive mole, choriocarcinoma, placental site trophoblastic tumor, and epithelioid trophoblastic tumor).

In some embodiments, the compound or product of the present application is administered as a prophylactic agent before tumor occurrence to prevent tumor occurrence or reduce the severity of subsequent tumors. In some embodiments, the compound or product of the present application is administered as a therapeutic agent after tumor occurrence to reduce the severity of the tumor disease, including its metastasis. In some embodiments, the compound or product of the present application is used as both a prophylactic agent and a therapeutic agent, administered continuously or intermittently before and after tumor occurrence.

A person skilled in the art can arbitrarily combine the foregoing technical solutions and technical features without departing from the inventive concept and scope of protection of the present disclosure. Other aspects of the present disclosure will be apparent to those skilled in the art from the disclosure herein.

### EXAMPLES

The present disclosure is further elaborated below in conjunction with specific examples. It should be understood that these examples are intended only to illustrate the present disclosure and not to limit the scope thereof. Those skilled in the art may make appropriate modifications and variations to the present disclosure, and such modifications and variations are within the scope of the present disclosure.

Where specific conditions are not indicated in the following examples, experimental methods can be carried out using conventional methods in the art, for example, by reference to Molecular Cloning: A Laboratory Manual (Third Edition, New York: Cold Spring Harbor Laboratory Press, 1989) or according to conditions recommended by suppliers. DNA sequencing methods are conventional in the art and can also be performed by commercial companies.

Unless otherwise indicated, percentages and parts are by weight. Unless otherwise defined, all professional and scientific terms used herein have the same meanings as familiar to those skilled in the art. In addition, any methods and materials similar or equivalent to those described herein can be applied in the methods of the present disclosure. The preferred embodiments and materials described herein are for illustrative purposes only.

### I. EXAMPLES OF SYNTHESIS AND CHARACTERIZATION OF COMPOUNDS

### Example I.1: Synthesis of Compound A (FPC-1)

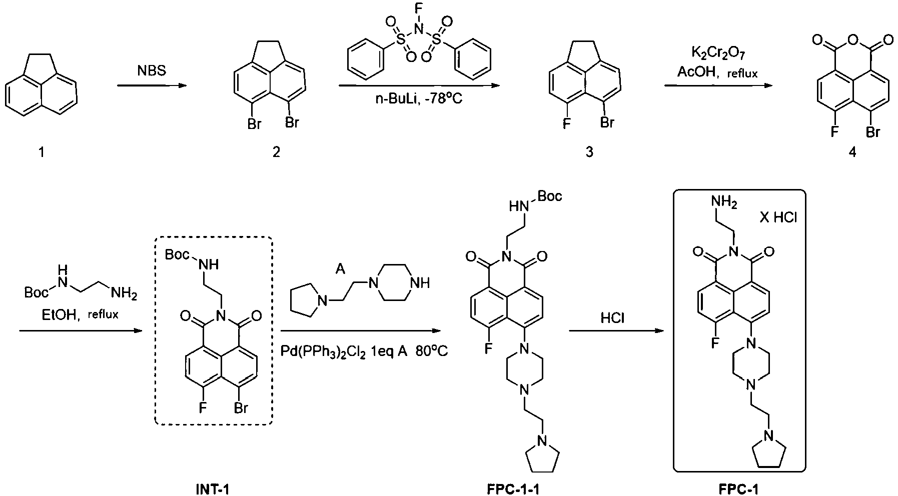

*eq: equivalent

### 1-1. Preparation of Compound 2

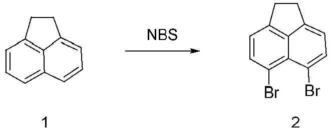

NBS (57.5 g, 320 mmol) was added to DMF (120 mL) and cooled to 0°C. Compound 1 (20 g, 130 mmol) was added, and the resulting suspension was stirred, allowed to warm naturally to room temperature, and stirred overnight. The reaction solution was filtered, and the filter cake was washed three times with ethanol. The collected filter cake was dried and recrystallized from EA to afford the pure product (10.1 g, off-white crystals).

¹H NMR (300 MHz, Chloroform-d) δ 7.79 (d, J = 7.4 Hz, 2H), 7.09 (d, J = 8.8 Hz, 2H), 3.30 (s, 4H).

### 1-2. Preparation of Compound 3

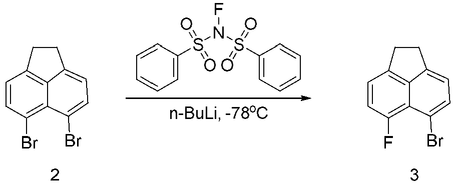

Compound 2 (6 g, 19.2 mmol) was dissolved in 300 mL of anhydrous THF and cooled to -80°C. Then, n-butyllithium (9.2 mL, 2.5 M) was added dropwise, and the reaction mixture was stirred at the same temperature for at least one hour. N-fluorobenzenesulfonimide (NFSI) (9 g, 28.8 mmol) in 100 mL of anhydrous THF was added slowly and then the mixture was stirred at -80°C for an additional 60 minutes. The reaction mixture was then allowed to warm to room temperature overnight, after which it was poured into an NH₄Cl solution. The aqueous phase was extracted with DCM (3 × 50 mL), and the combined organic phases were dried over MgSO₄. The solvent was removed under reduced pressure. The product was purified by column chromatography using n-hexane as the eluent to afford Compound 3 (4.1 g, white solid).

¹H NMR (300 MHz, Chloroform-d) δ 7.66 (d, J = 7.4 Hz, 1H), 7.21-7.09 (m, 3H), 3.35 (s, 4H).

### 1-3. Preparation of Compound 4

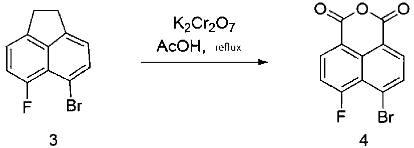

Compound 3 (4.5 g, 17.9 mmol) was added to a solution of potassium dichromate (24 g, 82.4 mmol) in glacial acetic acid (50 mL). The solution was heated to reflux for 16 hours, then poured into ice water. The resulting precipitate was collected by filtration and washed with water. The filter cake was air-dried, then added to DCM and refluxed for 30 minutes, filtered, and washed with DCM. The filter cake was added to DCM and refluxed for 15 minutes, filtered, and washed with DCM. All filtrates were combined and concentrated under vacuum to afford Compound **4** (4.1 g, crude, yellow solid).

### 1-4. Preparation of INT-1

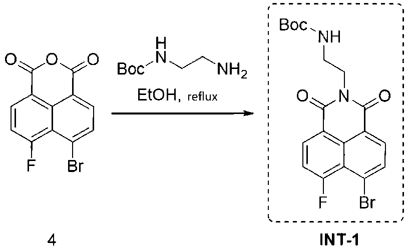

N-Boc-ethylenediamine (3.3 g, 20.8 mmol) was added to a solution of Compound **4** (4.1 g, 13.9 mmol) in EtOH (100 mL), and the mixture was heated to reflux for 1 hour. The reaction mixture was concentrated, and the resulting residue was purified by column chromatography (PE/EA = 5/1) to afford an off-white solid (2.8 g).

¹H NMR (300 MHz, Chloroform-d) δ 8.65 (dd, J = 8.2, 4.6 Hz, 1H), 8.43 (d, J = 8.0 Hz, 1H), 8.08 (d, J = 8.0 Hz, 1H), 7.50 (dd, J = 12.1, 8.1 Hz, 1H), 4.89 (s, 1H), 4.39-4.28 (m, 2H), 3.58-3.44 (m, 2H), 1.26 (s, 9H).

### 1-5. Preparation of FPC-1-1

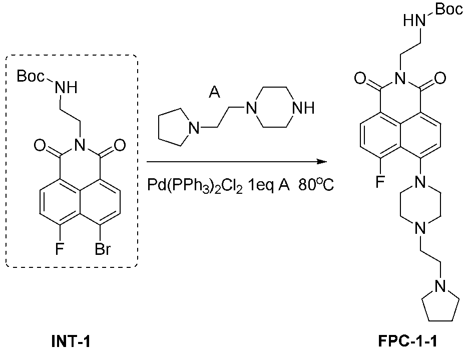

Cesium carbonate (5.55 g, 17 mmol) and 1-(2-(pyridin-2-one)ethyl)piperazine (1.05 g, 5.7 mmol) were added to a solution of **INT-1** (2.5 g, 5.7 mmol) in toluene (175 mL). Under argon atmosphere, bis(triphenylphosphine)palladium(II) chloride (400 mg, 0.57 mmol) was added to the mixture. The reaction was carried out at 80°C for 16 hours. The reaction solution was cooled to room temperature, water was added, and the mixture was stirred. The organic phase was separated, and the aqueous phase was extracted once with DCM. The combined organic phases were dried over anhydrous magnesium sulfate. After concentration under vacuum and column purification, the crude product was purified by Prep-TLC (DCM/MeOH = 10/1 + NH₃·H₂O) to afford 240 mg of pure product as a yellow solid.

¹H NMR (400 MHz, Chloroform-d) δ 8.56 (dd, J = 7.7, 4.3 Hz, 1H), 8.51 (d, J = 8.3 Hz, 1H), 7.34-7.27 (m, 1H), 7.17 (d, J = 8.2 Hz, 1H), 4.99 (s, 1H), 4.31 (s, 2H), 3.79-2.45 (m, 18H), 2.18 (s, 4H), 1.28 (s, 9H).

### 1-6. Preparation of FPC-1

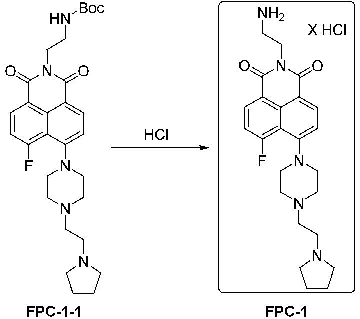

Compound **FPC-1-1** (240 mg, 0.44 mmol) was dissolved in 11 mL of DCM/MeOH (10/1). To the mixture was added 5 M HCl/1,4-dioxane (5 mL), and the mixture was stirred at room temperature for 2 hours. The mixture was concentrated under vacuum. The residue was dissolved in a small amount of methanol, precipitated with diethyl ether, and centrifuged. The precipitate was washed with diethyl ether, centrifuged, and dried under vacuum to afford 240 mg of the pure product as a yellow solid.

¹H NMR (300 MHz, methanol-d4) δ 8.67-8.58 (m, 2H), 7.56 (dd, J = 13.2, 8.3 Hz, 1H), 7.47 (d, J = 8.3 Hz, 1H), 4.49-4.42 (m, 2H), 4.02-3.69 (m, 10H), 3.69- 3.38 (m, 8H), 2.18 (s, 4H).

MS (ESI) m/z [M+H]⁺ = 440.3.

### Example 1.2: Synthesis of Compound B (FPC-2)

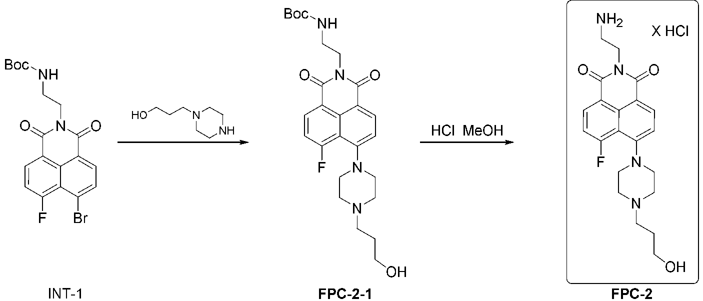

### 2-1. Preparation of FPC-2-1

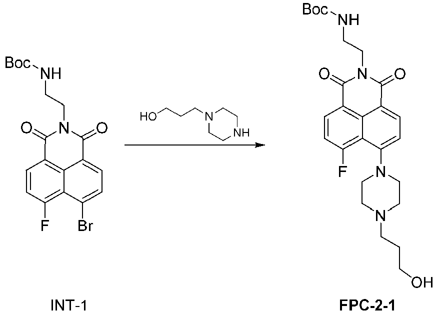

Into a single-neck flask were added 10 mL of ethylene glycol monomethyl ether, followed by **INT-1** (110 mg, 0.25 mmol) and 3-(piperazin-1-yl)propan-1-ol (44 mg, 0.3 mmol). The reaction mixture was stirred at 100°C. After 2 hours, the solvent was removed under vacuum, and the residue was purified by TLC to afford 20 mg of a yellow solid.

¹H NMR (400 MHz, Chloroform-d) δ 8.63 - 8.49 (m, 2H), 7.31 (dd, J = 12.8, 8.3 Hz, 1H), 7.18 (d, J = 8.3 Hz, 1H), 4.97 (s, 1H), 4.42 - 4.23 (m, 2H), 3.87 (t, J = 5.0 Hz, 2H), 3.60 - 3.28 (m, 8H), 2.92 (s, 4H), 1.89 (s, 2H), 1.29 (s, 9H).

### 2-2. Preparation of FPC-2

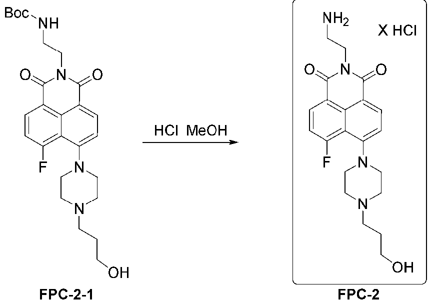

Compound **FPC-2-1** (20 mg, mmol) was dissolved in 2 mL of DCM/MeOH (1/1). To the mixture was added 5 M HCl/1,4-dioxane (2 mL), and the mixture was stirred at room temperature for 2 hours. The mixture was concentrated under vacuum. The residue was dissolved in a small amount of methanol, precipitated with diethyl ether, and centrifuged. The precipitate was washed with diethyl ether, centrifuged, and dried under vacuum to afford 5 mg of the pure product as a yellow solid.

¹H NMR (400 MHz, methanol-d4) δ 8.72-8.46 (m, 2H), 7.50 (ddd, J = 26.1, 12.4, 8.3 Hz, 2H), 4.45 (t, J = 5.6 Hz, 2H), 3.93-3.69 (m, 6H), 3.45 (dt, J = 15.0, 9.8 Hz, 5H), 3.34 (s, 3H), 2.07 (dt, J = 12.2, 6.1 Hz, 2H).

MS (ESI) m/z [M+H]⁺ = 401.47.

### Example 1.3: Synthesis of Compound C (FPC-3)

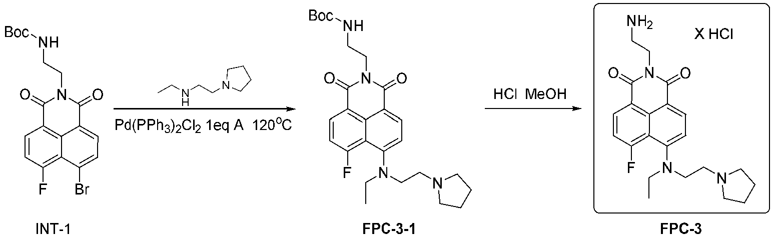

### 3-1. Preparation of FPC-3-1

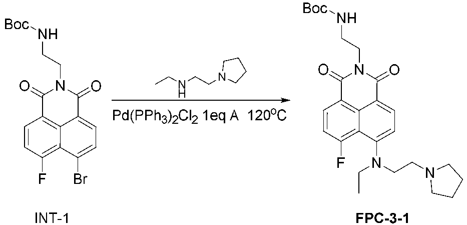

Cesium carbonate (1.78 g, 5.48 mmol) and N-ethyl-2-(pyrrolidin-1-yl)ethan-1-amine (286 mg, 2.01 mmol) were added to a solution of **INT-1** (800 mg, 1.82 mmol) in toluene (70 mL). Under argon atmosphere, bis(triphenylphosphine)palladium(II) chloride (128 mg, 0.18 mmol) was added to the mixture. The reaction was carried out at 80°C for 16 hours. The reaction solution was cooled to room temperature, water was added, and the mixture was stirred. The organic phase was separated, and the aqueous phase was extracted once with DCM. The combined organic phases were dried over anhydrous magnesium sulfate. After concentration under vacuum and column purification, the crude product was purified by Prep-TLC (DCM/MeOH = 10:1 + NH₃·H₂O) to afford a pure yellow solid (30 mg).

¹H NMR (400 MHz, Chloroform-d) δ 8.63-8.52 (m, 2H), 7.37-7.28 (m, 2H), 4.96 (s, 1H), 4.32 (t, J = 5.5 Hz, 2H), 3.99 (s, 2H), 3.93-3.75 (m, 2H), 3.49 (s, 2H), 3.44 (d, J = 6.8 Hz, 2H), 3.36-3.16 (m, 2H), 2.87-2.62 (m, 2H), 2.29-2.12 (m, 2H), 2.12-1.91 (m, 2H), 1.28 (s, 9H), 1.10 (t, J = 7.1 Hz, 3H).

### 3-2. Preparation of FPC-3

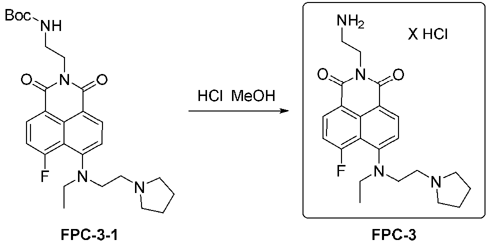

Compound FPC-3-1 (30 mg, 0.06 mmol) was dissolved in 4.4 mL of DCM/MeOH (10:1). To the mixture was added 5 M HCl/1,4-dioxane (2 mL), and the mixture was stirred at room temperature for 2 hours. The mixture was concentrated under vacuum. The residue was dissolved in a small amount of methanol, precipitated with diethyl ether, and centrifuged. The precipitate was washed with diethyl ether, centrifuged, and dried under vacuum to afford 25 mg of the pure product as a yellow solid.

¹H NMR (300 MHz, methanol-d4) δ 8.67-8.54 (m, 2H), 7.58-7.46 (m, 2H), 4.51-4.41 (m, 2H), 3.87-3.77 (m, 2H), 3.77-3.65 (m, 2H), 3.51 (q, J = 6.6 Hz, 4H), 3.34 (d, J = 1.8 Hz, 1H), 3.20-3.05 (m, 2H), 2.20-1.92 (m, 5H), 1.10 (t, J = 7.0 Hz, 3H).

MS (ESI) m/z [M+H]⁺ =399.47.

### Example 1.4: Synthesis of Compound D (FPC-5)

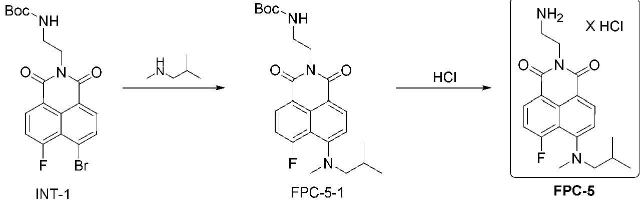

### 4-1. Preparation of FPC-5-1

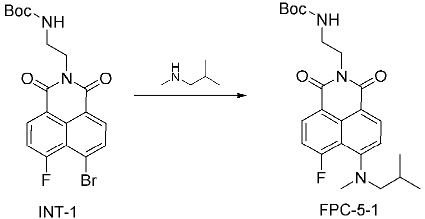

Cesium carbonate (460 mg, 1.41 mmol) and N,2-dimethylpropan-1-amine (49 mg, 0.56 mmol) were added to a solution of INT-1 (205 mg, 0.47 mmol) in toluene (20 mL). Under argon atmosphere, bis(triphenylphosphine)palladium(II) chloride (32 mg, 0.045 mmol) was added to the mixture. The reaction was carried out at 80°C for 16 hours. The reaction solution was cooled to room temperature, water was added, and the mixture was stirred. The organic phase was separated, and the aqueous phase was extracted once with DCM. The combined organic phases were dried over anhydrous magnesium sulfate. After concentration under vacuum and column purification, the crude product was purified by TLC (DCM/MeOH = 10/1 + NH₃·H₂O) to afford a pure yellow solid (10 mg).

¹H NMR (300 MHz, Chloroform-d) δ 8.56 (dd, J = 8.2, 4.9 Hz, 1H), 8.45 (d, J = 8.5 Hz, 1H), 7.24-7.18 (m, 1H), 7.07 (d, J = 8.5 Hz, 1H), 5.05 (s, 1H), 4.39-4.26 (m, 2H), 3.51 (dd, J = 11.9, 5.2 Hz, 2H), 3.20-3.13 (m, 1H), 3.05 (d, J = 4.0 Hz, 3H), 2.17-2.05 (m, 2H), 1.31 (s, 9H), 0.87 (d, J = 6.6 Hz, 6H).

### 4-2. Preparation of FPC-5

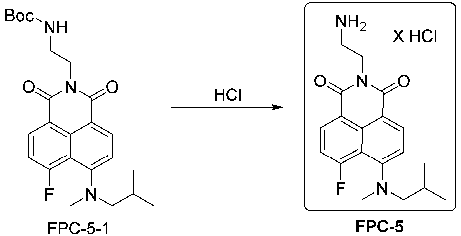

Compound **FPC-5-1** (10 mg, 0.022 mmol) was dissolved in 1.1 mL of DCM/MeOH (10:1). To the mixture was added 5 M HCl/1,4-dioxane (0.5 mL), and the mixture was stirred at room temperature for 2 hours. The mixture was concentrated under vacuum. The residue was dissolved in a small amount of methanol, precipitated with diethyl ether, and centrifuged. The precipitate was washed with diethyl ether, centrifuged, and dried under vacuum to afford 9 mg of the pure product as a yellow solid.

¹H NMR (400 MHz, methanol-d4) δ 8.61-8.52 (m, 1H), 8.44 (d, J = 8.3 Hz, 1H), 7.38 (dd, J = 12.5, 8.5 Hz, 1H), 7.25 (d, J = 8.4 Hz, 1H), 4.44 (t, J = 5.1 Hz, 2H), 3.22 (dd, J = 19.7, 7.1 Hz, 4H), 3.10 (d, J = 3.1 Hz, 3H), 2.16 (dp, J = 13.3, 7.2 Hz, 1H), 1.31 (d, J = 7.7 Hz, 1H), 0.87 (d, J = 6.4 Hz, 6H).

MS (ESI) m/z [M+H]⁺ =344.42.

### Example 1.5: Synthesis of Compound E (AIR-2)

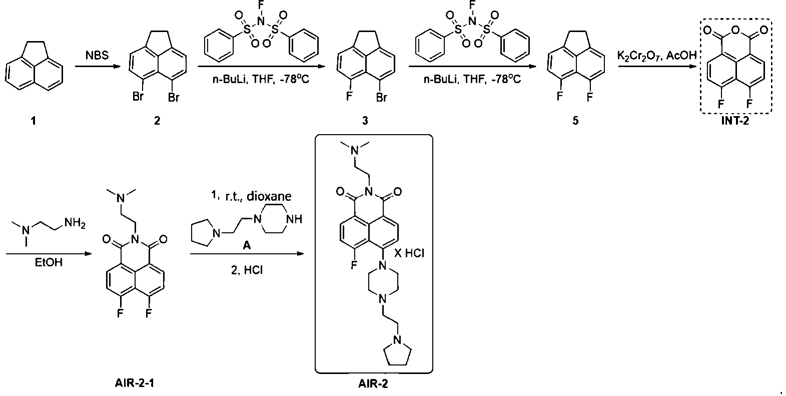

### 5-1. Preparation of Compound 5

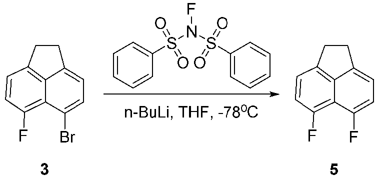

Compound 3 (31.90 g, 127.04 mmol) was dissolved in anhydrous THF (800 mL). The reaction system was purged with argon three times and then cooled to -78°C. n-BuLi (60.98 mL, 2.5 M/L, 152.45 mmol) was then added dropwise, and the reaction mixture was stirred at the same temperature for at least 10 minutes. N-fluorobenzenesulfonimide (NFSI) (60.09 g, 190.57 mmol) in 200 mL of anhydrous THF was added dropwise. After stirring at -78°C for 1 hour, the reaction mixture was allowed to warm naturally to room temperature and stirred overnight. The reaction mixture was quenched with an NH₄Cl solution and then extracted twice with EA. The combined organic layers were washed with water and brine, dried over Na₂SO₄, and filtered. The filtrate was concentrated to obtain a crude product, which was added to DCM and stirred. The resulting precipitate was filtered, and the filter cake was washed twice with DCM. All filtrates were combined and concentrated under vacuum to afford a crude product, which was then purified by column chromatography (PE) to obtain Compound **5** as a white solid (15.42 g, 63.82% yield).

¹H NMR (300 MHz, Chloroform-d) δ 7.16 (d, J = 7.6 Hz, 2H), 7.12-7.04 (m, 2H), 3.37 (s, 4H).

### 5-2. Preparation of INT-2

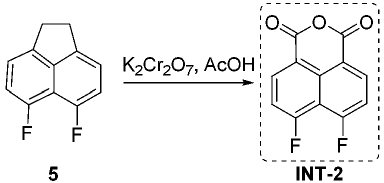

Compound 5 (15.42 g, 81.08 mmol) was added to a solution of potassium dichromate (109.72 g, 372.96 mmol) in glacial acetic acid (220 mL). The solution was stirred at 80°C for 12 hours. The reaction mixture was quenched with EA and water, and extracted twice with EA. The combined organic layers were washed with water and brine, dried over Na₂SO₄, and filtered. The filtrate was concentrated to obtain a crude product, to which PE was added and the mixture was sonicated to disperse. The resulting precipitate was filtered and dried to afford crude INT-2 as a brownish-yellow solid (10.55 g), which was used without further purification.

### 5-3. Preparation of AIR-2-1

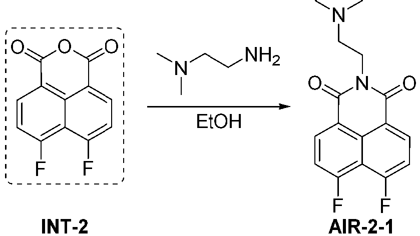

**INT-2** (5 g, 21.35 mmol) was dissolved in EtOH (120 mL). The reaction system was purged with argon three times and then heated to 50°C. N¹,N¹-dimethylethane-1,2-diamine (2.79 mL, 25.62 mmol) was added, and the mixture was heated to 80°C (reflux) for 4 hours. TLC showed completion of the reaction. The reaction mixture was concentrated, and the obtained residue was purified by column chromatography (DCM: MeOH = 30:1 to 10:1) to afford **AIR-2-1** as a brownish-yellow solid (1.81 g, 27.86% yield). MS (ESI), m/z: 305.4 [M+H]⁺.

¹H NMR (300 MHz, Chloroform-d) δ 8.65 (t, J = 2.5 Hz, 1H), 8.62 (t, J = 2.5 Hz, 1H), 7.47-7.39 (m, 2H), 4.38 (t, J = 6.7 Hz, 2H), 2.89-2.81 (m, 2H), 2.49 (s, 6H).

### 5-4. Preparation of AIR-2

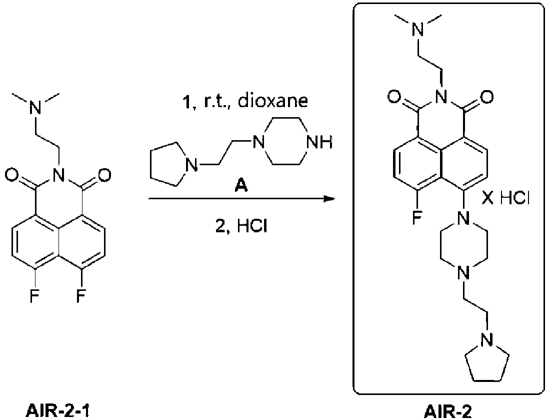

**AIR-2-1** (1.81 g, 5.95 mmol) was dissolved in 1,4-dioxane (150 mL). The reaction system was purged with argon three times. 1-(2-pyridinone-ethyl)piperazine (Compound A) (1.20 g, 6.54 mmol) was added, and the mixture was stirred at room temperature overnight. The reaction mixture was concentrated, and the obtained residue was purified by column chromatography (DCM: MeOH = 15:1 to 5:1) to afford the pure product dissolved in DCM. Then, 5 M HCl/1,4-dioxane (1.5 mL) was added and stirring was continued. The mixture was precipitated with diethyl ether. The resulting precipitate was filtered, washed with diethyl ether, and dried to afford **AIR-2** as a yellow solid (1.21 g). MS (ESI), m/z: 468.47 [M+H]⁺.

¹H NMR (300 MHz, D₂O) δ 8.36 (dd, J = 8.3, 4.7 Hz, 1H), 8.29 (d, J = 8.3 Hz, 1H), 7.40 (dd, J = 13.1, 8.3 Hz, 1H), 7.27 (d, J = 8.4 Hz, 1H), 4.42 (t, J = 6.0 Hz, 2H), 3.90-3.54 (m, 12H), 3.47 (t, J = 6.0 Hz, 2H), 3.38-3.12 (m, 4H), 2.99 (s, 6H), 2.21-2.02 (m, 4H).

### Example 1.6: Synthesis of Compound F (AIR-3)

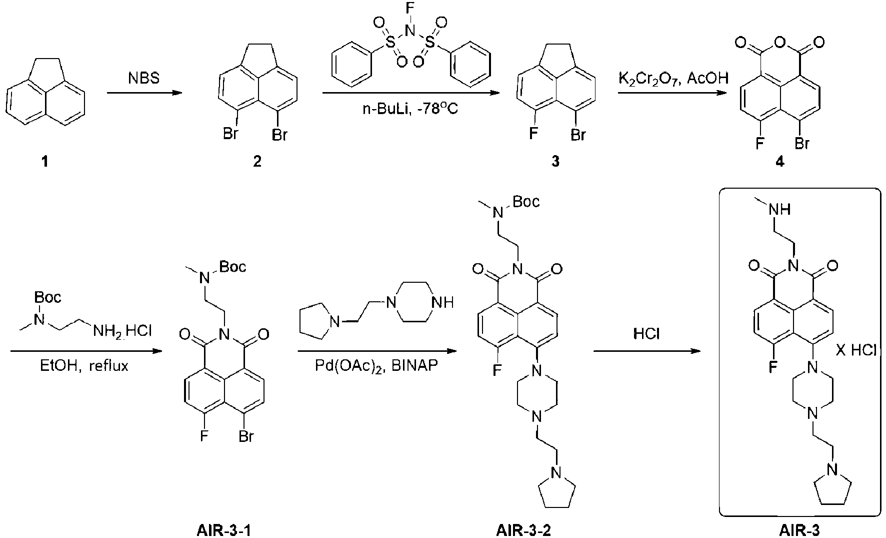

### 6-1. Preparation of Compound 2

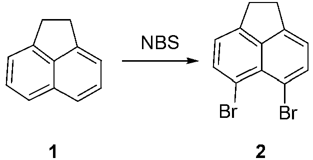

NBS (499.72 g, 2.81 mol) was added to DMF (1000 mL) and stirred until most of the solid dissolved. The reaction system was purged with argon twice and then cooled to 9°C. Compound 1 (176 g, 1.14 mol) was added. After stirring at 9°C for 2 hours, the reaction mixture was allowed to warm naturally to room temperature and stirred over the weekend. The reaction solution was filtered, and the filter cake was washed twice with PE/EA = 1/1. The dried filter cake was collected and recrystallized from EA to afford pure Compound 2 as off-white crystals (102.57 g, 28.80% yield).

¹H NMR (300 MHz, Chloroform-d) δ 7.79 (d, J = 7.4 Hz, 2H), 7.09 (d, J = 8.8 Hz, 2H), 3.30 (s, 4H).

### 6-2. Preparation of Compound 3

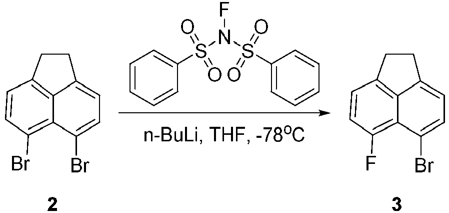

Compound 2 (50 g, 160.26 mmol) was dissolved in anhydrous THF (950 mL). The reaction system was purged with argon three times and then cooled to -78°C. n-BuLi (76.92 mL, 2.5 M/L, 192.31 mmol) was then added dropwise, and the reaction mixture was stirred at the same temperature for at least 20 minutes. N-fluorobenzenesulfonimide (NFSI) (75.8 g, 240.38 mmol) in 250 mL of anhydrous THF was added dropwise. After stirring at -78°C for 1.5 hours, the reaction mixture was allowed to warm naturally to room temperature and stirred overnight. The reaction mixture was quenched with an NH₄Cl solution and then extracted twice with EA. The combined organic layers were washed with water and brine, dried over Na₂SO₄, and filtered. The filtrate was concentrated to obtain a crude product, which was added to DCM and stirred. The resulting precipitate was filtered, and the filter cake was washed twice with DCM. All filtrates were combined and concentrated under vacuum to afford a crude product, which was then purified by column chromatography (PE:EA = 50:1) to obtain Compound 3 as a white solid (31.90 g, 79.27% yield).

¹H NMR (300 MHz, Chloroform-d) δ 7.66 (d, J = 7.4 Hz, 1H), 7.21 - 7.09 (m, 3H), 3.35 (s, 4H).

### 6-3. Preparation of Compound 4

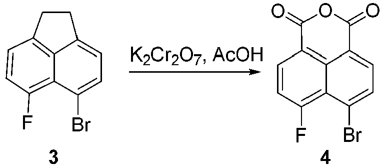

Compound 3 (9.29 g, 37.00 mmol) was added to a solution of potassium dichromate (50.07 g, 170.19 mmol) in glacial acetic acid (100 mL). The solution was heated to reflux for 16 hours and then poured into ice water. The resulting precipitate was collected by filtration and washed with water. The filter cake was air-dried, then added to DCM and refluxed for 30 minutes, filtered, and washed with DCM. The filter cake was added to DCM and refluxed for 15 minutes, filtered, and washed with DCM. All filtrates were combined and concentrated to afford crude Compound 4 (6.72 g), which was used without further purification.

### 6-4. Preparation of AIR-3-1

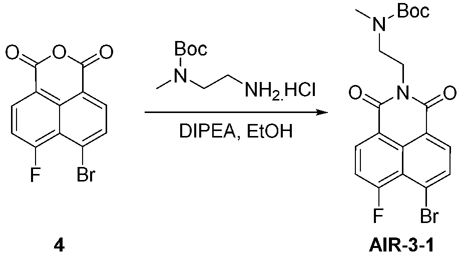

To a solution of Compound **4** (400 mg, 1.36 mmol) in EtOH (20 mL) were added DIPEA (358.48 µL, 2.17 mmol) and tert-butyl (2-aminoethyl)(methyl)carbamate hydrochloride (428.46 mg, 2.03 mmol). The reaction system was purged with argon five times and then stirred at 80°C (reflux) for 2 hours. TLC showed completion of the reaction. The reaction mixture was concentrated, and the obtained residue was purified by column chromatography (PE:EA = 10:1 to 4:1) to afford **AIR-3-1** as a yellow solid (244 mg, 39.88% yield).

### 6-5. Preparation of AIR-3-2

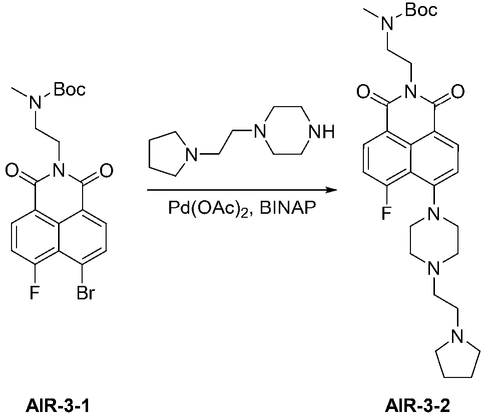

To a solution of **AIR-3-1** (244 mg, 540.68 mmol) in toluene (15 mL) were added NaOt-Bu (103.92 mg, 1.08 mol), 1-(2-pyridinone-ethyl)piperazine (118.92 mg, 648.81 mmol), and BINAP (101.00 mg, 162.20 mmol). The reaction system was purged with argon five times, then Pd(OAc)₂ (24.28 mg, 108.14 mmol) was added. The reaction system was purged with argon three times, and the reaction mixture was stirred at 90°C under argon atmosphere for 12 hours. The reaction mixture was quenched with water and extracted twice with EA. The combined organic layers were washed with water and brine, dried over Na₂SO₄, and filtered. The filtrate was concentrated to obtain a crude product, which was purified by column chromatography (DCM: MeOH = 100:1 to 10:1, with NH₃·H₂O) and Prep-TLC (DCM: MeOH = 10:1, with NH₃·H₂O) to afford **AIR-3-2** (40 mg, 13.36% yield).

¹H NMR (300 MHz, Chloroform-d) δ 8.56 (dd, J = 8.2, 4.7 Hz, 1H), 8.51 (d, J = 8.3 Hz, 1H), 7.29 (dd, J = 7.7, 5.1 Hz, 1H), 7.15 (d, J = 8.3 Hz, 1H), 4.36-4.29 (m, 2H), 3.62-3.55 (m, 2H), 3.45-3.11 (m, 6H), 2.95 (s, 3H), 2.82-2.62 (m, 10H), 1.82-1.89 (m, 4H), 1.14 (s, 9H).

### 6-6. Preparation of AIR-3

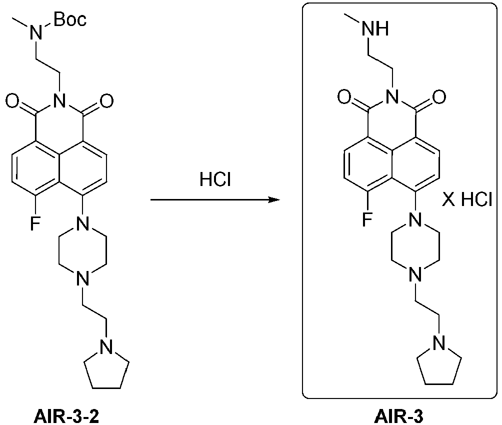

A solution of **AIR-3-2** (40 mg, 72.24 mmol) in DCM/MeOH = 10/1 (3 mL) was purged with argon three times, then 5 M HCl/1,4-dioxane (1.5 mL) was added. The resulting mixture was stirred at room temperature for 1.5 hours. TLC showed completion of the reaction. The reaction mixture was precipitated with diethyl ether and centrifuged. The precipitate was washed with diethyl ether, centrifuged, and dried to afford a crude product (33 mg). The crude product was further purified by Prep-TLC (DCM: MeOH = 20:1, with NH₃·H₂O) to afford **AIR-3** as a yellow solid (10 mg). MS (ESI), m/z: 454.58 [M+H]⁺. HPLC 92.439% (220nm), HPLC 96.712% (254nm).

¹H NMR (300 MHz, D₂O) δ 8.35 (dd, J = 8.4, 4.7 Hz, 1H), 8.28 (d, J = 8.3 Hz, 1H), 7.39 (dd, J = 13.2, 8.5 Hz, 1H), 7.26 (d, J = 8.3 Hz, 1H), 4.39-4.31 (m, 2H), 3.86-3.62 (m, 14H), 3.37-3.31 (m, 2H), 3.25-3.15 (m, 2H), 2.70 (s, 3H), 2.24-2.13 (m, 2H), 2.08-1.98 (m, 2H).

### Example II. Activity Testing

### Example 11.1: Activation of the hnRNPA2B1 and TBK1-IRF3 Pathways by Test Compounds in Tumor Cells

A549 lung cancer cells (cell density: 1 × 10⁵ cells per well) were stimulated with test compound E (prepared as in Example I.5, dissolved in physiological saline) at a concentration of 20 µM for 30 minutes. The subcellular localization of hnRNPA2B1 was detected by immunofluorescence, and the activation of TBK1 and IRF3 was detected by SDS-PAGE electrophoresis and immunoblotting.

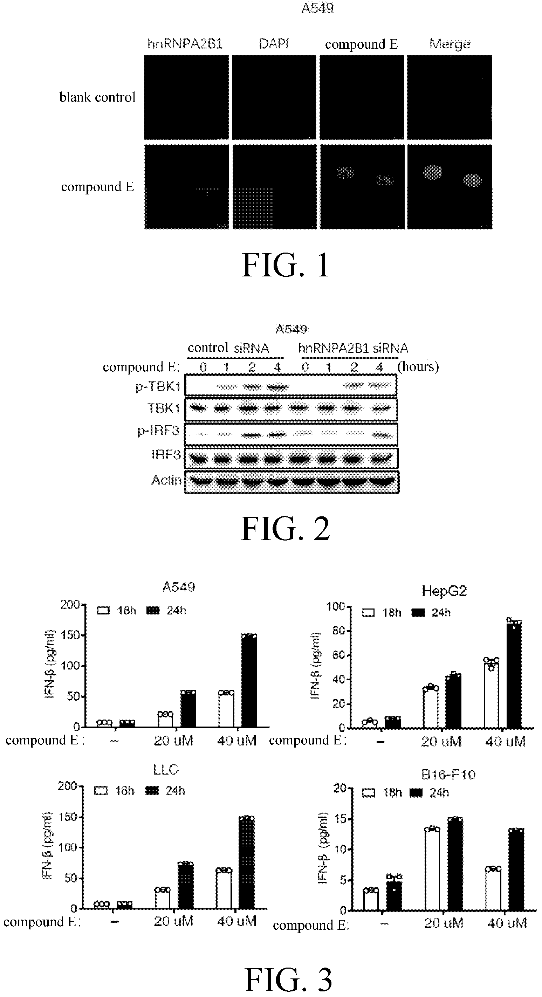

The activation of hnRNPA2B1, TBK1, and IRF3 is shown in Figures 1 and 2.

The results showed that test compound E significantly activated the nuclear export of hnRNPA2B1 and the activation of downstream TBK1 and IRF3 in tumor cells. Furthermore, other compounds prepared in Example I were tested under the same conditions, and similar results were obtained.

These results demonstrate that the test compounds activate the hnRNPA2B1 pathway and act as an hnRNPA2B1 agonist.

### Example II.2: Broad Induction of Type I Interferon in Multiple Tumor Types by the Test Compounds

A549 (non-small cell lung cancer), HepG2 (hepatocellular carcinoma), LLC (Lewis lung carcinoma), and B16F10 (melanoma) tumor cells (cell density: 1 × 10⁴ cells per well) were stimulated with test compound E at concentrations of 20 and 40 µM. Cell culture supernatants were collected at 18 and 24 hours post-stimulation, and IFN-β protein levels were detected by ELISA.

The activation of type I interferon by the test compound is shown in Figure 3.

The results showed that test compound E significantly activated type I interferon expression in multiple cell types. Furthermore, other compounds prepared in Example I were tested under the same conditions, and similar results were obtained.

These results demonstrate that the test compounds induce type I interferon production by activating the hnRNPA2B1 pathway and are biologically active hnRNPA2B1 agonists. Therefore, these novel compounds are defined as hnRNPA2B1 agonists in the present disclosure.

### Example II.3: Inhibitory Effect of the Test Compounds on Proliferation of Multiple Tumor Cell Types

A549 (non-small cell lung cancer), HepG2 (hepatocellular carcinoma), LLC (Lewis lung carcinoma), and B16F10 (melanoma) tumor cells (cell density: 1 × 10⁴ cells per well) were stimulated with test compound E at a concentration of 20 µM. After 24 and 48 hours of stimulation, the cells were lysed, and the effect of compound E on tumor cell viability was detected by CCK-8 assay.

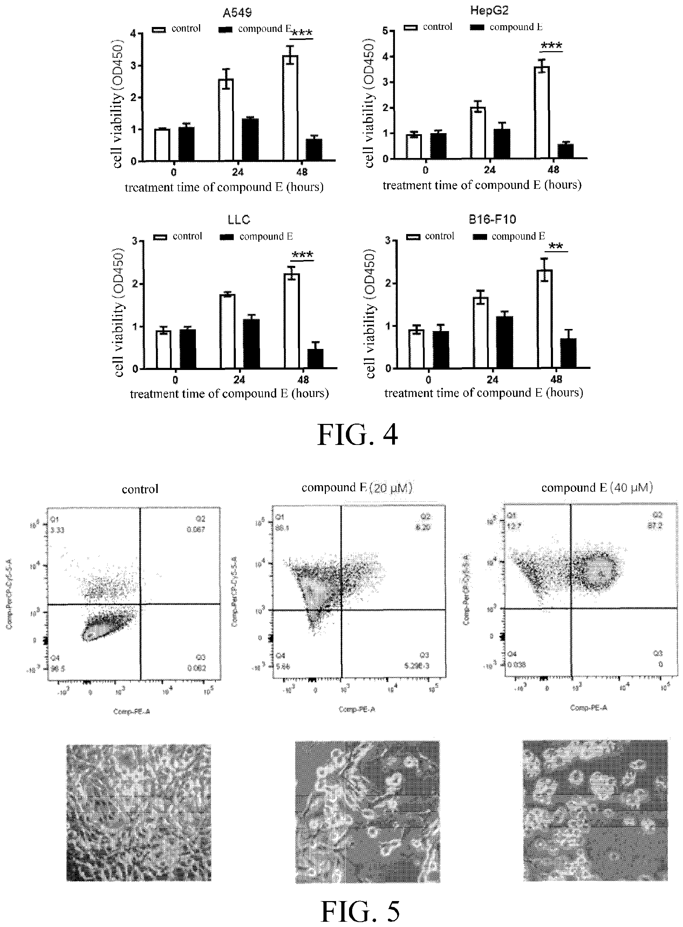

The inhibitory effect of test compound E on tumor cells is shown in Figure 4.

The results showed that test compound E significantly inhibited the proliferation of multiple tumor cell types. Furthermore, other compounds prepared in Example I were tested under the same conditions, and similar results were obtained.

These results demonstrate that the test compounds significantly inhibit tumor cell proliferation by targeting hnRNPA2B1.

### Example II.4: Cytotoxic Effect of the Test Compounds on Multiple Tumor Cell Types

LLC (Lewis lung carcinoma) tumor cells (cell density: 1 × 10⁵ cells per well) were stimulated with test compound E at concentrations of 20 and 40 µM. Cell morphology was observed under a microscope at 36 hours post-stimulation, and after cell digestion, the cytotoxic effect of compound E on tumor cells was detected by flow cytometry.

The cytotoxic effect of test compound E on tumor cells is shown in Figure 5.

The results showed that test compound E significantly induced tumor cell apoptosis. Furthermore, other compounds prepared in Example I were tested under the same conditions, and similar results were obtained.

These results demonstrate that the test compounds effectively kill tumor cells by targeting hnRNPA2B1.

### Example II.5: Inhibition of LLC Tumor Proliferation and Promotion of Survival of Tumor-Bearing Mice induced by hnRNPA2B1 Agonists

Mice (6- to 8-week-old male C57BL/6J mice) were subcutaneously inoculated with 5 × 10⁵ LLC tumor cells. Starting from day 10 post-inoculation, compound E (15 mg/kg mouse body weight) was administered via tail vein injection once every four days for a total of four doses. Tumor growth was continuously observed, with tumor volume calculated as (length × width²)/2, and mouse survival was monitored.

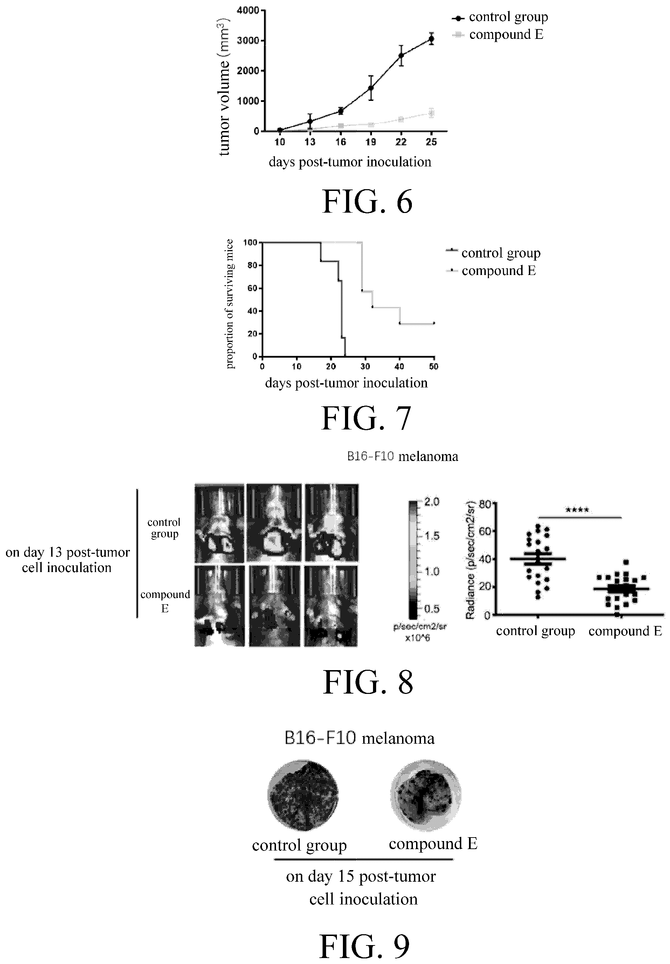

The test results are shown in Figures 6 and 7.

The results showed that the hnRNPA2B1 agonist compound E inhibited LLC tumor proliferation and promoted the survival of tumor-bearing mice. Furthermore, other compounds prepared in Example I were tested under the same conditions, and similar results were obtained.

These results indicate that hnRNPA2B1 agonists effectively exert anti-tumor effects.

### Example II.6: Inhibition of B16F10 Tumor Proliferation and Promotion of Survival of Tumor-Bearing Mice by hnRNPA2B1 Agonists

Mice (6- to 8-week-old male C57BL/6J mice) were subcutaneously inoculated with 5 × 10⁵ B16-F10 melanoma cells. Starting from day 10 post-inoculation, compound E (15 mg/kg mouse body weight) was administered via tail vein injection once every four days for a total of four doses. Tumor growth was continuously observed, with tumor volume calculated as (length × width²)/2.

The results showed that the hnRNPA2B1 agonist inhibited B16F10 tumor proliferation. Furthermore, other compounds prepared in Example I were tested under the same conditions, and similar results were obtained.

These results indicate that hnRNPA2B1 agonists effectively exert anti-tumor effects.

### Example II.7: Inhibition of Experimental Lung Metastasis of B16F10 Tumors and Promotion of Survival of Tumor-Bearing Mice by hnRNPA2B1 Agonists

Mice (6- to 8-week-old male C57BL/6J mice) were injected via the tail vein with 5 × 10⁴ B16-F10 melanoma cells. Starting from day 6 post-injection, compound E (10 mg/kg mouse body weight) was administered via tail vein injection once every three days for a total of three doses. On day 13 post-administration, lung tumor conditions were observed and quantitatively compared using a small animal *in vivo* imaging system. On day 15, the mice were euthanized, and the lungs were isolated to observe tumor conditions.

The test results are shown in Figures 8 and 9.

The results showed that the hnRNPA2B1 agonist inhibited experimental lung metastasis of B16F10 tumors. Furthermore, other compounds prepared in Example I were tested under the same conditions, and similar results were obtained.

These results indicate that hnRNPA2B1 agonists effectively exert anti-tumor metastasis effects.

### Biological Evaluation

The compounds of the present disclosure are defined as hnRNPA2B1 agonists by the following criteria: (i) binding to the hnRNPA2B1 protein, as demonstrated by high affinity binding of the compound at concentrations as low as 20 µM to the hnRNPA2B1 protein via Biacore assays; (ii) cellular analysis demonstrating that the compounds broadly induce higher levels of IFN-β at concentrations as low as 20 µM.

### (i) Biacore T200 Detection of Binding Between the compound and hnRNPAB1

The following were used:
Series S CM5 chip; amino coupling reagents: 1-(3-dimethylaminopropyl)-3-ethylcarbodiimide (EDC) and N-hydroxysuccinimide (NHS)
Coupling buffer: 10 mM sodium acetate, pH 4.0, pH 4.5
Running buffer: 10× PBS-P+
Analytical grade DMSO, deionized water (0.22 µm membrane filtered)
hnRNPA2B1 recombinant protein (concentration > 0.5 mg/mL, total protein at least 20 µg)
Test compound: stock solution concentration > 20 mM, volume > 30 µL, purity > 90%, dissolved in 100% DMSO
Other consumables: 96-well plate and sealing film, 1.5 mL centrifuge tubes without lids, Type 2 rubber bottle caps

### Experimental Procedure:

1. Place the running buffer (1× PBS) and waste bottle in the designated positions, and place the chip.
2. Protein coupling. Select the "immobilization program" in the Wizard template, check "flow cell" 2 or 4, select "amine" as the method, choose "specify contact time and flow rate" for high coupling, enter a "contact time" of 900 seconds, with a flow rate of 10 µL/min.
3. Prepare the required protein solution according to the instructions. Dilute the protein to the required volume with pH 4.0 sodium acetate to a final concentration of 20 µg/mL. Place 100 µL of EDC, 100 µL of NHS, and 140 µL of ethanolamine in the corresponding positions.
4. The system automatically coats the chip surface with the target coupling amount of hnRNPA2B1 protein and automatically generates a coupling report.
5. Prepare the running buffer for interaction. The running buffer for small molecule samples is 1× PBS-P+ containing 5% DMSO: dilute 105 mL of 10× PBS-P+ to 1 L with deionized water to prepare 1.05× PBS-P+.
   Solvent calibration stock solution containing 4.5% DMSO: 9.5 mL of (1.05× PBS-P+) + 0.45 mL DMSOSolvent calibration stock solution containing 5.8% DMSO: 9.5 mL of (1.05× PBS-P+) + 0.58 mL DMSO
   Running buffer containing 5% DMSO: 950 mL of (1.05× PBS-P+) + 50 mL DMSO
   Dilute 10 mM small molecule stock solution with DMSO-free 1.05× PBS-P+ buffer: 0.7 µL of small molecule stock solution + 6.3 µL DMSO + 133 µL 1.05× PBS-P+ buffer, total volume 140 µL, final concentration 50 µM. Add to a 96-well plate and seal with a sealing film.

After detection, determine the binding affinity using Kinetics or Affinity mode.

### (ii) Total cellular RNA can be extracted using TRIzol or a rapid RNA extraction kit.

Rapid RNA extraction from 24-well plate cells (approximately 2-3 × 10⁵ cells/well): Remove the culture medium, add 500 µL of RA2 lysis buffer to each well, lyse by pipetting multiple times, and transfer to the inner sleeve of the designated adsorption tube. Centrifuge at 12,000 rpm for 1 minute at room temperature. Discard the filtrate, add 500 µL of wash buffer, and centrifuge at 12,000 rpm for 1 minute at room temperature. Repeat the wash once. Discard the filtrate, return the inner sleeve, and centrifuge at 12,000 rpm for 2 minutes at room temperature without adding wash buffer. Transfer the inner sleeve to a new 1.5 mL centrifuge tube, add 25 µL of Elution Buffer to the center of the membrane, let stand for 5 minutes at room temperature, and centrifuge at 12,000 rpm for 1 minute at room temperature. The resulting filtrate is the RNA. Determine the RNA concentration using Nanodrop One.

qRT-PCR: After concentration determination for each sample, take 1 µg of total RNA and use ReverTra Ace^{®} qRT-PCR RT Master Mix to reverse transcribe the RNA into cDNA. The reverse transcription system and reaction conditions are as follows:

### Reverse Transcription Reaction System (20 µL system):

| Components | Volume |
|---|---|
| 5×RT Master Mix | 4 µL |
| RNA | 1 µg |
| DEPC water | added to 20 µL |

### Reverse Transcription Reaction Conditions (20 µL system)

| Temperature | Time |
|---|---|
| 37°C | 15 min |
| 50°C | 5 min |
| 98°C | 5 min |
| 4°C | ∞ |

Dilute the reverse-transcribed cDNA sample with 60 µL of deionized water, mix well, and centrifuge briefly. The primer sequence information used for qRT-PCR is shown in the table. Use SYBR Green Realtime PCR Master Mix for relative quantitative detection of gene mRNA levels.

### qRT-PCR Reaction System (20 µL system):

| Components | Volume |
|---|---|
| 2×SYBR Green Realtime PCR Master Mix | 10 µL |
| cDNA template | 4 µL |
| F-primer | 0.5 µL |
| R-primer | 0.5 µL |
| DNase/RNase free H₂O | 5 µL |

### qRT-PCR Reaction Conditions

| Temperature | Time | Notes |
|---|---|---|
| 95°C | 1 min | |
| 95°C | 10 s | 40 cycles |
| 60°C | 20 s | |
| 72°C | 20 s | |
| 95°C | 1 min | |
| 65°C | 1 min | |
| 95°C | 15 s | |

### qRT-PCR prime

| Gene | Primer Sequence |
|---|---|
| *IFNB* Forward | GCTTGGATTCCTACAAAGAAGCA |
| *IFNB* Reverse | ATAGATGGTCAATGCGGCGTC |
| *ACTIN* Forward | CATGTACGTTGCTATCCAGGC |
| *ACTIN* Reverse | CTCCTTAATGTCACGCACGAT |

All documents mentioned in the present disclosure are incorporated by reference into this application as if each individual document was separately incorporated by reference. Furthermore, it should be understood that, after reading the above teachings of the present disclosure, those skilled in the art may make various changes or modifications to the present disclosure, and such equivalent forms also fall within the scope defined by the appended claims of the present application.

## Claims

1. A fused cyclic compound represented by Formula (I), a cis-trans isomer, an enantiomer, a diastereomer, a racemate, a solvate, a hydrate, or a pharmaceutically acceptable salt or a prodrug thereof, 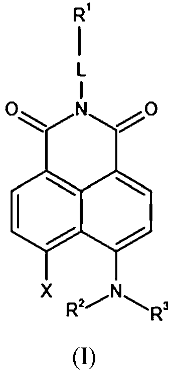 wherein:
L is -(CH₂)n-, and n is an integer from 0 to 6;
X is halogen;
R¹ is -NR⁴R⁵, wherein R⁴ and R⁵ are each independently H or C₁₋₆ alkyl;
R² and R³ are each independently H or C₁₋₆ alkyl;
or R² and R³, together with the N atom to which they are attached, form a 5- to 8-membered heterocycloalkyl group containing 1 to 3 heteroatoms selected from nitrogen, oxygen, or sulfur;
said C₁₋₆ alkyl and said 5- to 8-membered heterocycloalkyl group are unsubstituted or substituted with one or more substituents selected from the group consisting of: hydroxy, amino, halogen, C₁₋₆ alkyl, C₂₋₆ alkenyl, C₂₋₆ alkynyl, C₁₋₆ alkoxy, hydroxy C₁₋₆ alkyl, C₁₋₆ alkylamino, C₃₋₆ cycloalkyl, C₃₋₆ cycloalkyl C₁₋₆ alkyl, 3- to 6-membered heterocycloalkyl, 3- to 6-membered heterocycloalkyl C₁₋₆ alkyl, 6- to 10-membered aryl, 6- to 10-membered aryl C₁₋₆ alkyl, or 5- to 10-membered heteroaryl and 5- to 10-membered heteroaryl C₁₋₆ alkyl.

2. The compound, the cis-trans isomer, the enantiomer, the diastereomer, the racemate, the solvate, the hydrate, or the pharmaceutically acceptable salt or the prodrug thereof according to claim 1,
wherein X is F or Cl;
L is -(CH₂)n-, wherein n is 1, 2 or 3;
R⁴ and R⁵ are each independently H, methyl or ethyl;
R² and R³ are each independently C₁₋₃ alkyl, wherein said C₁₋₃ alkyl is unsubstituted or substituted with a 3- to 8-membered heterocycloalkyl group containing 1 to 2 heteroatoms selected from nitrogen, oxygen or sulfur;
or R² and R³, together with the N atom to which they are attached, form a 5- to 8-membered heterocycloalkyl group containing two nitrogen atoms, wherein said 5- to 8-membered heterocycloalkyl group is substituted with a 3- to 8-membered heterocycloalkyl C₁₋₆ alkyl group or a hydroxy C₁₋₆ alkyl group.

3. The compound, the cis-trans isomer, the enantiomer, the diastereomer, the racemate, the solvate, the hydrate, or the pharmaceutically acceptable salt or the prodrug thereof according to claim 1,
wherein X is F;
L is -(CH₂)n-, wherein n is 2;
R⁴ and R⁵ are each independently H, methyl, or ethyl;
R² and R³ are each independently C₁₋₃ alkyl, wherein said C₁₋₃ alkyl is unsubstituted or substituted with a 4- to 6-membered heterocycloalkyl group containing one nitrogen heteroatom;
or R² and R³, together with the N atom to which they are attached, form a piperazinyl group, wherein said piperazinyl group is substituted with a 4- to 6-membered heterocycloalkyl C₁₋₆ alkyl group or a hydroxy C₁₋₆ alkyl group.

4. The compound, the cis-trans isomer, the enantiomer, the diastereomer, the racemate, the solvate, the hydrate, or the pharmaceutically acceptable salt or the prodrug thereof according to claim 1,
wherein X is F;
L is -(CH₂)n-, wherein n is 2;
R⁴ and R⁵ are each independently H, methyl, or ethyl;
R² and R³ are each independently methyl, ethyl or isopropyl, wherein said C₁₋₃ alkyl is unsubstituted or substituted with a pyrrolidinyl group;
or R² and R³, together with the N atom to which they are attached, form a piperazinyl group, wherein said piperazinyl group is substituted with a pyrrolidinylethyl group or a hydroxyethyl group.

5. The compound, the cis-trans isomer, the enantiomer, the diastereomer, the racemate, the solvate, the hydrate, or the pharmaceutically acceptable salt or the prodrug thereof according to claim 1, wherein the compound has the formula (II): 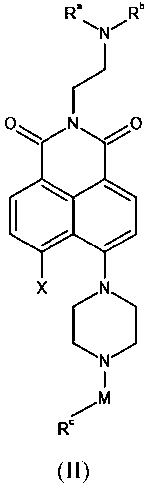
wherein, R^{a} and R^{b} are each independently H or C₁₋₆ alkyl;
M is -(CH₂)n-, wherein n is 1, 2 or 3;
X is F or Cl;
R^{c} is hydroxy, amino, or a 3- to 8-membered heterocycloalkyl group containing 1 to 2 heteroatoms selected from nitrogen, oxygen or sulfur; more preferably, R^{c} is hydroxy or a 4- to 6-membered heterocycloalkyl group containing one nitrogen heteroatom; even more preferably, R^{c} is hydroxy or pyrrolidinyl; most preferably, R^{c} is hydroxy or 1-pyrrolidinyl.

6. The compound, the cis-trans isomer, the enantiomer, the diastereomer, the racemate, the solvate, the hydrate, or the pharmaceutically acceptable salt or the prodrug thereof according to claim 1, wherein the compound is selected from the following compounds: 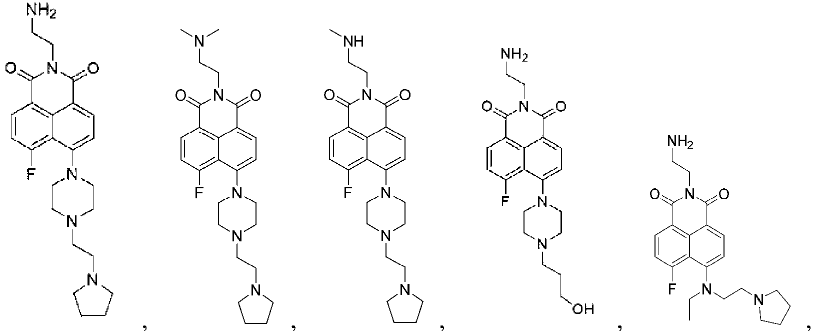 or 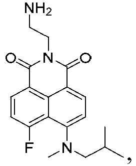 or a hydrochloride salt thereof.

7. A product comprising the compound according to any one of claims 1 to 6, and a pharmaceutically or immunologically acceptable carrier or excipient.

8. The product according to claim 7, wherein the product is selected from: a pharmaceutical composition, a drug, a kit, or a medicine kit; and/or
wherein the product is used as a product for inducing type I interferon production in a subject and/or used for preventing and/or treating a tumor in a subject.

9. Use of the compound according to any one of claims 1 to 6 in the preparation of a product for inducing type I interferon production in a subject or a product for preventing and/or treating a tumor in a subject.

10. The use according to claim 9, wherein the subject is a human or a non-human mammal, such as a non-human primate (e.g., gorilla, chimpanzee), a pet (e.g., pet cat, dog, guinea pig, rabbit), a livestock animal (e.g., cow, horse, donkey, mule, camel, swine, caprine, chicken, duck, goose, rabbit, deer, mink, otter, musk deer, etc.); and/or
wherein the production of type I interferon is mediated by hnRNPA2B1; and/or
wherein the prevention and/or treatment of the tumor in the subject can benefit from the *in vivo* production or an increased level of type I interferon; and/or
wherein the tumor is one or more tumors selected from the group consisting of: solid tumors, such as liver cancer, lung cancer, melanoma, breast cancer, ovarian cancer, prostate cancer, kidney cancer, intestinal cancer, head and neck cancer, skin cancer, bladder cancer, pancreatic cancer; non-solid tumors, such as hematological tumors (e.g., leukemia), nervous system tumors (e.g., glioma); preferably, non-small cell lung cancer, liver cancer, Lewis lung carcinoma, melanoma, breast cancer, colon cancer; and/or
wherein the product is selected from: a pharmaceutical composition, a medicament, a kit, or a medicine kit; and/or
wherein the product is used as a product for inducing type I interferon production in a subject and/or used for preventing and/or treating a tumor in a subject.
